(19) Europäisches Patentamt / European Patent Office / Office européen des brevets

(11) **EP 4 574 056 A1**

(12) **DEMANDE DE BREVET EUROPEEN**

(43) Date de publication:
**25.06.2025 Bulletin 2025/26**

(21) Numéro de dépôt: **24221846.9**

(22) Date de dépôt: **19.12.2024**

(51) Classification Internationale des Brevets (IPC):
*A61B 8/08* ($^{(2006.01)}$)

(52) Classification Coopérative des Brevets (CPC):
**A61B 8/5207; A61B 8/0841; A61B 8/485; A61B 8/5223**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité: **21.12.2023 FR 2314789**

(71) Demandeurs:
• **SUPERSONIC IMAGINE**
**13290 Aix-en-Provence (FR)**
• **Centre National de la Recherche Scientifique (CNRS)**
**75016 Paris (FR)**
• **Ecole Supérieure de Physique et de Chimie Industrielles de la Ville de Paris**
**75005 Paris (FR)**
• **Université Gustave Eiffel - ESIEE Paris**
**77454 Marne la Vallée Cedex 2 (FR)**

(72) Inventeurs:
• **Le Ber, Arthur**
**75016 PARIS (FR)**

• **Goicoechea, Antton**
**75016 PARIS (FR)**
• **Lambert, William**
**13290 Aix-en-Provence (FR)**
• **Aubry, Alexandre**
**75016 PARIS (FR)**
• **Jia, Xiaoping**
**75005 PARIS (FR)**
• **Tourin, Arnaud**
**75005 PARIS (FR)**
• **Fink, Mathias**
**75005 PARIS (FR)**
• **Etaix, Nicolas**
**13290 Aix-en-Provence (FR)**
• **Rotter, Stefan**
**A-1040 VIENNA (AT)**
• **Rachbauer, Lukas**
**A-1040 VIENNA (AT)**

(74) Mandataire: **RVDB Nantes**
**2, rue Crucy**
**CS 60515**
**44005 Nantes Cedex 1 (FR)**

(54) **PROCÉDÉ POUR CARACTÉRISER UN OBJET CIBLE DANS UN MILIEU**

(57) La présente divulgation concerne un procédé pour caractériser un objet cible dans un milieu. Le procédé comprend : obtenir (S1) une matrice de référence $F(q)$ associée à l'objet cible, mesurer (S2) une matrice de réflexion $R$ associé au milieu sondé, et projeter (S3) la matrice de réflexion $R$ sur la matrice de référence $F(q)$ pour estimer une probabilité $P(q)$ que l'objet soit dans un état $q$.

**Fig.1**

S1 : obtenir une matrice de référence F(q) ou un dictionnaire des matrices de référence associée à l'objet cible

S1a (option) : mesurer expérimentalement une première matrice de référence et/ou un premier jeu de matrices de référence

S1b (option) : constituer la matrice de référence F(q) ou le dictionnaire de matrices de référence de manière entièrement synthétique

S1c (option) : optimiser la matrice de référence F(q) ou le dictionnaire des matrices de référence

S2 : obtenir une matrice de réflexion R du milieu

S3 : projeter la matrice de réflexion R sur la matrice de référence F(q) pour estimer une probabilité P(q) que l'objet soit dans un état q

S3a (option) : appliquer un filtre matriciel à la matrice de réflexion R

EP 4 574 056 A1

**Description**

**Technique antérieure**

L'échographie, c'est-à-dire l'imagerie par ultrasons, offre une méthode non invasive pour visualiser la structure interne d'un matériau, d'un animal, d'un corps humain ou de certaines de ses parties (par exemple les organes).

Des exemples de différents modes d'imagerie par ultrasons peuvent comprendre un mode B (c'est-à-dire un mode de luminosité), un mode Doppler ou un mode ShearWave® Elastographie (c'est-à-dire un mode d'élastographie par ondes de cisaillement).

En échographie, un réseau de transducteurs piézoélectriques est typiquement placé en vis-à-vis du milieu que l'on souhaite imager. Ces éléments peuvent émettre/recevoir des ondes ultrasonores de manière indépendante sur une large bande spectrale. A partir d'une série d'insonifications du milieu par ce réseau, les échos rétrodiffusés par les hétérogénéités du milieu sont enregistrés par ces mêmes transducteurs ou par d'autres. Les signaux ainsi enregistrés sont ensuite stockés dans une matrice de réponses. Cette matrice peut être acquise pour différents types d'ondes incidentes.

Une séquence d'acquisition basique consiste à émettre chaque onde incidente avec un élément à la fois et, pour chaque émission, à enregistrer le champ réfléchi par le milieu sur tous les éléments du réseau. Cette base canonique, ou base des transducteurs, a été utilisée pour la première fois pour décrire le principe du retournement temporel itératif, cf. la méthode « DORT » décrite par : Claire Prada, Mathias Fink, « Eigenmodes of the time reversal operator: A solution to sélective focusing in multiple-target media, », Wave Motion, Volume 20, Issue 2, 1994, Pages 151-163, ISSN 0165-2125. Cette base canonique peut être utilisée dans le domaine du contrôle non destructif, où elle est appelée "full matrix capture".

En outre, il existe des technologies d'imagerie par ultrasons qui utilisent la composition d'ondes planes cohérentes pour l'ultrasonographie et l'élastographie transitoire à très haute fréquence d'images, cf. Montaldo G, Tanter M, Bercoff J, Benech N, Fink M. Cohérent plane-wave compounding for very high frame rate ultrasonography and transient elastography. IEEE Trans Ultrason Ferroelectr Freq Control. 2009 Mar;56(3):489-506. doi: 10.1109/TUFFC.2009.1067. PMID: 19411209.

Dans ce contexte, FR3114159 décrit un procédé de caractérisation ultrasonore d'un milieu comprenant une étape de génération d'une série d'ondes ultrasonores incidentes, une étape de génération d'une matrice de réflexion expérimentale $\mathbf{R}_{ui}(t)$ définie entre la base d'émission ($\mathbf{i}$) en entrée et une base de réception (u) en sortie, une étape de détermination d'une matrice de réflexion focalisée $\mathbf{R}_{rr} = [R(r_{in}, r_{out}, \delta t)]$ du milieu entre un transducteur virtuel d'entrée ($TV_{in}$) calculé à partir d'une focalisation en entrée de la matrice de réflexion expérimentale et un transducteur virtuel de sortie ($TV_{out}$) calculé à partir d'une focalisation en sortie de la matrice de réflexion expérimentale, les réponses du transducteur de virtuel de sortie ($TV_{out}$) étant prises à un instant temporel décalé d'un délai additionnel $\delta t$ par rapport à un instant temporel des réponses du transducteur virtuel d'entrée ($TV_{in}$). Cette matrice de réflexion focalisée joue un rôle pivot dans le développement d'une échographie matricielle à des fins de quantification et de correction des problèmes d'aberrations et de diffusion multiple en imagerie ultrasonore.

**Exposé de la divulgation**

Le procédé et le système décrits dans le présent document concernent des technologies de détection, de localisation et de caractérisation d'un objet cible, notamment des objets difficiles à imager, par exemple des objets en milieu diffusant. La présente description s'applique notamment, mais non exclusivement, à l'imagerie médicale ou vétérinaire pour la localisation et la caractérisation de cibles dans les milieux biologiques (par exemple des micro-calcifications, des bulles, les aiguilles ou les marqueurs de lésion en imagerie ultrasonore). Elle peut également s'appliquer à la détection de défauts utiles pour un contrôle non destructif dans l'industrie. Bien que les démonstrations expérimentales présentées dans ce document soient réalisées en réflexion dans le domaine de l'imagerie ultrasonore, cette divulgation peut s'appliquer également en transmission à tous les domaines de la physique des ondes (par exemple des technologies de radar, microscopie, sismologie) pour lesquels un ou plusieurs réseaux multi-éléments permettent de mesurer la matrice de réponse d'un milieu en réflexion et/ou en transmission de ce dernier.

En particulier, il peut être souhaitable de détecter de manière plus fiable la position et/ou l'orientation d'un objet cible dans un milieu étudié, dit aussi région d'intérêt. Par exemple, si l'objet cible est un dispositif médical (par exemple un marqueur) implanté dans un corps humain, il est souhaitable de localiser avec précision sa position dans le tissu humain, par exemple dans le cadre d'une intervention chirurgicale telle qu'une ablation de tumeur ou masse suspecte.

Selon un exemple plus général, il peut être possible de caractériser un objet cible dans un milieu, par exemple, non seulement pour localiser sa position, mais aussi pour obtenir d'autres informations sur l'objet ou son environnement température, pression, ...), par exemple sa forme et/ou la structure de l'objet, la taille de l'objet, la composition de l'objet, et/ou suivre l'évolution de ces paramètres la nature, structure, composition en fonction de temps.

Selon une autre modalité, l'état de l'objet peut être utilisé pour caractériser le milieu environnant (pression, température, propriétés mécaniques, etc.), la réponse de l'objet dépendant également du milieu environnant la région d'intérêt.

Comme expliqué plus en détail ci-dessous, les techniques conventionnelles (par exemple mode B de l'imagerie par ultrasons) ne permettent souvent pas une détection ou caractérisation précise d'un objet cible, par exemple en raison de la présence d'un milieu diffusant générant un "speckle" ultrasonore. Le "speckle" peut être considéré comme un profil granulaire, qui peut varier d'un endroit à l'autre du milieu. Le "speckle" peut s'expliquer par la formation cohérente de l'écho à partir de nombreux petits diffuseurs au sein du milieu. Certains objets sont également difficiles à imager voire indétectables avec les techniques d'imagerie conventionnelle en raison de leur géométrie ou de leurs propriétés acoustiques ou mécaniques.

Ainsi, un procédé pour caractériser un objet cible dans un milieu est fourni selon un exemple de réalisation particulier de la divulgation. Le procédé comprend :
obtenir une matrice de référence $F(q)$ associée à l'objet cible (par exemple associé à un état q de l'objet cible), obtenir une matrice de réflexion $R$ du milieu, et projeter la matrice de réflexion $R$ sur la matrice de référence $F(q)$ pour estimer une probabilité $P(q)$ que l'objet soit dans un état $q$.

Par conséquent, ainsi que divulgué dans la présente demande, étant donné que l'on obtient une matrice de réflexion du milieu (et donc de l'objet cible contenu), et que cette matrice de réflexion peut être projetée sur une matrice de référence de l'objet cible (c'est-à-dire qui "décrit" l'objet cible), l'objet cible peut être caractérisé de manière fiable et précise dans le support, indépendamment de tout facteur de perturbations du milieu.

En d'autres termes, la matrice de référence peut être conçue, prédéterminée et utilisée pour rechercher dans un milieu d'intérêt et/ou détecter l'objet cible de manière plus fiable et plus précise.

Donc la matrice de référence $F(q)$ peut permettre de caractériser l'objet cible.

Par exemple, le milieu peut être un tissu mammaire comprenant un marqueur. Un tissu mammaire typiquement provoque un speckle ultrasonore. La présence d'un objet cible (par exemple un marqueur) est difficilement décelable sur une image ultrasonore conventionnelle de mode B. En revanche, l'utilisation d'une matrice de référence $F(q)$ associé à l'objet cible permet une détection avec un excellent contraste et une localisation avec une précision bien inférieure à la limite de diffraction du milieu.

Selon un exemple de réalisation particulier, l'objet cible comprend au moins un parmi : un élément prédéfini du milieu, tel qu'un tissu du milieu, comme par exemple une fibre musculaire, et un objet externe inséré dans le milieu, tel qu'un dispositif médical, par exemple un marqueur de type biomarqueur, tout ou partie d'un dispositif de biopsie, une aiguille ou une sonde.

Selon un exemple de réalisation particulier, l'état q de l'objet comprend au moins un paramètre parmi : la position relative et/ou l'orientation relative de l'objet dans le milieu, l'orientation de l'objet dans le milieu, la forme et/ou la structure (initiale et/ou actuelle) de l'objet, la taille de l'objet, la composition de l'objet, des paramètres intensifs locaux du milieu environnant, tels que la température, la pression, la composition, des propriétés mécaniques et/ou la concentration d'un composé du milieu environnant.

Selon un exemple de réalisation particulier, l'état $q$ de l'objet peut être mesuré à différents instants et peut ainsi être caractérisé de manière dynamique.

Par exemple, l'obtention de la matrice de réflexion peut comprendre d'obtenir des matrices de réflexion mesurées à différents instants t. Chacune des matrices de réflexion peut être projetée sur la matrice de référence pour mesurer la dynamique de l'état de l'objet.

En conséquence, la probabilité estimée que l'objet cible se trouve dans un état particulier peut fournir des informations sur les caractéristiques de l'objet ou sur son environnement. Par exemple, si l'on estime avec une forte probabilité que l'objet a une taille particulière et se trouve dans une position particulière, cette taille et cette position peuvent caractériser en partie ou totalement l'objet.

L'état de la cible peut également être exprimé sous la forme d'une matrice $Q$ :
Chaque colonne $q_i$ (sous forme vectorielle) de la matrice $Q$ peut correspondre à un paramètre d'état de l'objet cible, par exemple $q_1$ : Position, $q_2$ : Orientation, etc.

Selon un exemple de réalisation particulier, obtenir une matrice de référence $F(q)$ comprend l'obtention d'un dictionnaire de matrices de référence $F(q)$ associé à l'objet cible.

Le dictionnaire matriciel permet par exemple d'exploiter la signature matricielle d'un objet cible pour le caractériser précisément, par exemple pour le détecter avec un excellent contraste dans un milieu qui génère du speckle lorsqu'il est insonifié par exemple par des ondes ultrasonores.

Selon un exemple de réalisation particulier, projeter la matrice de réflexion $R$ comprend : projeter la matrice de réflexion $R$ sur l'ensemble des matrices de référence $F(q)$ pour estimer une probabilité $P(q)$ que l'objet soit dans un état $q$.

Selon un exemple de réalisation particulier, projeter la matrice de réflexion $R$ comprend l'application d'un filtre matriciel à la matrice de réflexion $R$.

Selon un exemple de réalisation particulier, le filtre matriciel comprend un ou plusieurs produits scalaires normalisés entre la matrice de réflexion $R$ et l'ensemble des matrices de référence $F(q)$, ou le filtre matriciel comprend un modèle d'intelligence artificielle.

Également, le produit scalaire normalisé peut être un corrélateur et/ou une fonction mathématique. Le résultat de cette fonction mathématique peut être la probabilité estimée.

Le modèle d'intelligence artificielle est par exemple un réseau neuronal.

Selon un exemple de réalisation particulier, la matrice de réflexion **R** est une matrice de réflexion exprimée dans la base focalisée ou dans une autre base, telle que des bases ondes planes, des transducteurs, etc. Les données d'entrée pour le modèle d'intelligence artificielle peuvent comprendre la matrice de réflexion **R** et la matrice de référence **F(q)**. Le résultat du modèle d'intelligence artificielle peut comprendre la probabilité estimée. Le modèle peut être pré-entraîné à la tâche d'estimer la probabilité.

Selon un exemple de réalisation particulier, les matrices de référence **F(q)** du dictionnaire dépend d'un état de référence de l'objet cible ou de son environnement, l'état optionnellement au moins un parmi : la position, l'orientation, la forme, la structure, la taille, la composition et/ou les paramètres intensifs locaux du milieu environnant comme la température, la pression ou la concentration d'un composé.

Selon un exemple de réalisation particulier, obtenir le dictionnaire des matrices de référence **F(q)** comprend : une mesure (par exemple de type expérimental) d'une première matrice de référence correspondant à une matrice de réflexion $R_0(q_0)$ pour un premier état $q_0$ de l'objet cible, et/ou une mesure (par exemple de type expérimental) d'un premier jeu de matrices de référence pour un premier jeu d'états **q** de l'objet cible et/ou associées à plusieurs états **q** de l'objet cible. principalement, une matrice de référence peut correspondre à ou peut être une matrice de réflexion selon la présente divulgation. Toutefois, la matrice de référence a une fonction spécifique, à savoir servir de référence (par exemple dans le cadre d'un dictionnaire). Toutefois, la manière dont elle est obtenue peut-être la même, par exemple dans le cadre d'une mesure.

La différence entre la matrice de référence et la matrice de réflexion peut donc résider dans le contexte dans lequel elle est mesurée. Alors qu'une matrice de référence selon la présente divulgation peut être mesurée lors d'une expérience, afin d'obtenir une référence d'un objet cible, la matrice de réflexion selon la présente divulgation peut être mesurée sur un support particulier (par exemple un patient) pendant le déploiement.

Par conséquent, la ou les matrices de référence peuvent être obtenues lors de la préparation de la méthode ou du système et être stockées à l'avance, la matrice de réflexion peut être obtenue "à la volée" (par exemple en temps réel ou quasi réel) pendant le déploiement de la méthode ou du système. La projection de la matrice de réflexion sur la matrice de référence peut également être effectuée "à la volée" (par exemple en temps réel ou quasi réel).

Selon un exemple de réalisation particulier, obtenir le dictionnaire des matrices de référence **F(q)** comprend : le calcul numérique d'une deuxième matrice de référence pour un deuxième état **q** de l'objet cible, à partir de la première matrice de référence, par exemple mesurée pour un état $q_0$ de l'objet cible.

Selon un exemple de réalisation particulier pour lequel l'état **q** est la position **r** de l'objet cible, la deuxième matrice de référence est simulée numériquement à partir de la première matrice de référence mesurée pour une première position $r_0$ de l'objet par une translation virtuelle de l'objet cible dans une nouvelle position **r,** cette opération étant optionnellement réalisée dans une base focalisée, la base des transducteurs, ou dans la base d'ondes planes.

La deuxième matrice de référence peut être simulée numériquement, par exemple en utilisant un modèle de propagation des ondes depuis un réseau de transducteurs jusqu'à l'objet cible et un modèle de diffusion des ondes par l'objet cible.

Le choix de la base, dans laquelle la translation virtuelle devrait être réalisée, peut être dicté par une minimisation du coût de calcul induit.

Selon un exemple de réalisation particulier, le dictionnaire de matrices de référence comprend la première et la deuxième matrices de référence.

Selon un exemple de réalisation particulier, le dictionnaire des matrices de référence est adapté/ajusté/optimisé en utilisant au moins l'une des méthodes suivantes :

- adaptation du filtre matriciel en fonction de la première matrice de référence afin de rendre le filtre matriciel plus spécifique à l'objet cible recherché par rapport au milieu environnant ;
- optimisation du spectre fréquentiel du dictionnaire pour améliorer le contraste entre l'objet cible et un bruit associé au milieu ;

- et construction du dictionnaire à partir d'un estimateur $R_0^{-1}(q_0)$ de la matrice inverse de la première matrice de référence $R_0(q_0)$.

Selon un exemple de réalisation particulier, la première matrice de référence $R_0(q_0)$ mesurée et/ou la matrice de réflexion **R** est obtenue par une méthode d'imagerie et/ou d'échographie ultrasonore.

Selon un exemple de réalisation particulier, obtenir la première matrice de référence mesurée et/ou la matrice de réflexion comprend : - une étape de génération d'une série d'ondes incidentes (USin) dans un milieu, au moyen d'un réseau (10) de transducteurs (11), ladite série d'ondes incidentes étant une base d'émission (**i**) ; et - une étape de génération d'une matrice de réflexion expérimentale $\mathbf{R}_{ui}(t)$ définie entre la base d'émission (**i**) en entrée et une base de réception (**u**) (c'est à dire une base de transducteurs) en sortie pour un temps d'écho t; - une étape de détermination d'une matrice de réflexion focalisée $\mathbf{R}_{rr} = [R(\mathbf{r}_{in}, \mathbf{r}_{out}, \delta t)]$ étant une base focalisée, la matrice de réflexion focalisée $\mathbf{R}_{rr}$ comprenant des réponses du milieu $R(\mathbf{r}_{in}, \mathbf{r}_{out}, \delta t)$ entre un transducteur virtuel d'entrée (TVin) de position spatiale $\mathbf{r}_{in}$ et un transducteur virtuel de sortie (TVout) de position spatiale $\mathbf{r_{out}}$, les réponses du transducteur de virtuel de sortie (TVout) étant prises à un instant temporel décalé d'un délai additionnel $\delta t$ par rapport à un instant temporel des réponses du transducteur virtuel d'entrée (TVin).

Les ondes peuvent comprendre des ondes ultrasonores.

La matrice de réflexion focalisée peut être obtenue numériquement par un processus de formation de voies - c'est à dire par « beamforming ».

Selon un exemple de réalisation particulier, obtenir une matrice de référence $\mathbf{F}(\mathbf{q})$ comprend : la constitution d'un dictionnaire de matrices de référence de manière entièrement synthétique en simulant numériquement une première matrice de référence d'un réflecteur virtuel (par exemple un miroir de réflectivité d'orientation $\alpha$ et de taille $\ell_c$.) pour un premier état $\mathbf{q}_0$ , et en calculant numériquement une deuxième matrice de référence pour un nouvel état $\mathbf{q}$ de l'objet cible à partir de la première matrice de réflexion $\mathbf{R_0}(\mathbf{q_0})$.

Donc le dictionnaire de matrices de référence peut également être constitué de manière entièrement synthétique, c'est-à-dire sans mesure expérimentale.

La présente divulgation concerne également un procédé pour reconnaître un objet cible dans un milieu, le procédé comprenant un procédé selon l'un quelconque des aspects précédents.

La présente divulgation concerne également un procédé pour générer une cartographie d'un milieu comprenant un objet cible, comprenant un procédé selon l'un quelconque des aspects précédents, et/ou générer la cartographie, notamment en fonction de l'état estimé de l'objet cible.

Selon un exemple de réalisation particulier, la cartographie du milieu comprend une image du milieu (par exemple obtenue par un procédé prédéfini) et une identification de l'objet cible superposée, dans laquelle l'identification de l'objet cible est déterminée par un procédé selon l'un des aspects précédents.

Par exemple, un point à l'emplacement de l'objet cible ou une image (qui peut être une image schématique ou une photo) de l'objet cible est superposée à l'image du milieu à l'endroit correspondant.

La présente divulgation se rapporte également à un programme d'ordinateur comprenant des instructions qui, lorsque le programme est exécuté par un ordinateur, conduisent celui-ci à mettre en oeuvre le procédé selon l'un quelconque des aspects précédents.

La présente divulgation selon un mode de réalisation particulier se rapporte également à un système pour caractériser un objet cible dans un milieu. Le système comprend un processeur configuré pour :

- obtenir une matrice de référence $\mathbf{F}(\mathbf{q})$ associé à l'objet cible,
- obtenir une matrice de réflexion $\mathbf{R}$ du milieu,
- projeter la matrice de réflexion $\mathbf{R}$ sur la matrice de référence $\mathbf{F}(\mathbf{q})$ pour estimer une probabilité $P(\mathbf{q})$ que l'objet soit dans un état $\mathbf{q}$.

Le système peut avoir des fonctionnalités qui correspondent aux opérations du procédé selon la présente divulgation.

Par exemple, le système peut être un ordinateur qui peut être associé à un système d'imagerie ultrasonore. Il est également possible que le système soit un système d'imagerie ultrasonore.

Les caractéristiques et avantages de la divulgation apparaitront à la lecture de la description qui va suivre, donnée uniquement à titre d'exemple non limitatif et faite en référence aux figures annexées. Notamment, les exemples illustrés dans les figures peuvent être combinés sauf incohérence notoire.

**Brève description des figures**

D'autres caractéristiques et avantages de la présente divulgation ressortiront de la description des exemples de réalisation particuliers et non limitatifs de la présente divulgation ci-après, en référence aux figures 1 à 12 annexées, sur lesquelles :

[Fig. 1] montre schématiquement un procédé pour caractériser un objet cible dans un milieu selon des exemples de la présente divulgation.

[Fig. 2] montre un dessin schématique d'un système d'imagerie ultrasonore selon des exemples de la présente divulgation.

[Fig. 3] montre schématiquement le principe d'une méthode pour décomposition de l'opérateur retournement temporel selon un exemple.

[Fig. 4] montre schématiquement un diagramme résumant les différentes étapes de la méthode de projection matricielle à partir d'une mesure de calibration de la matrice de réflexion de l'objet cible selon des exemples de la présente divulgation.

[Fig. 5] montre schématiquement une preuve de concept selon une première expérience comprenant une imagerie matricielle de sphères d'acier dans des milieux granulaires fortement diffusants selon des exemples de la présente divulgation.

[Fig. 6] montre schématiquement un dispositif et une méthode pour obtenir une matrice de référence $R_0(q_0)$ mesurée expérimentalement dans le contexte de la première expérience selon des exemples de la présente divulgation. Elle décrit également l'information contenue dans cette matrice suivant ses composantes spatiales et temporelles.

[Fig. 7] montre schématiquement une méthode de détection et localisation d'un marqueur de lésions dans le speckle ultrasonore selon une deuxième expérience, selon des exemples de la présente divulgation.

[Fig. 8] montre schématiquement un exemple de cartographie matricielle des fibres musculaires d'un mollet, dans lequel le dictionnaire de matrices de référence est constitué de manière entièrement synthétique selon la présente divulgation

[Fig. 9] montre schématiquement la translation virtuelle de la position de l'objet cible selon des exemples de la présente divulgation.

[Fig. 10] montre schématiquement la transmission et réception d'une onde plane associée à un angle d'incidence à partir duquel les données doivent être filtrées dans la matrice $R(q=q_0)$ selon des exemples de la présente divulgation.

[Fig. 11] montre schématiquement des cartes de vraisemblance de position pour chaque cible résultant de la projection matricielle selon des exemples de la présente divulgation.

[Fig. 12] montre schématiquement un spectre des valeurs singulières $\sigma_i$ des matrices $R(q_i=q_0)$ associées aux sphères selon des exemples de la présente divulgation.

## Description des modes de réalisation

Sur les différentes figures, fournies à titre d'illustration, les mêmes références numériques désignent des éléments identiques ou similaires sauf incohérence notoire.

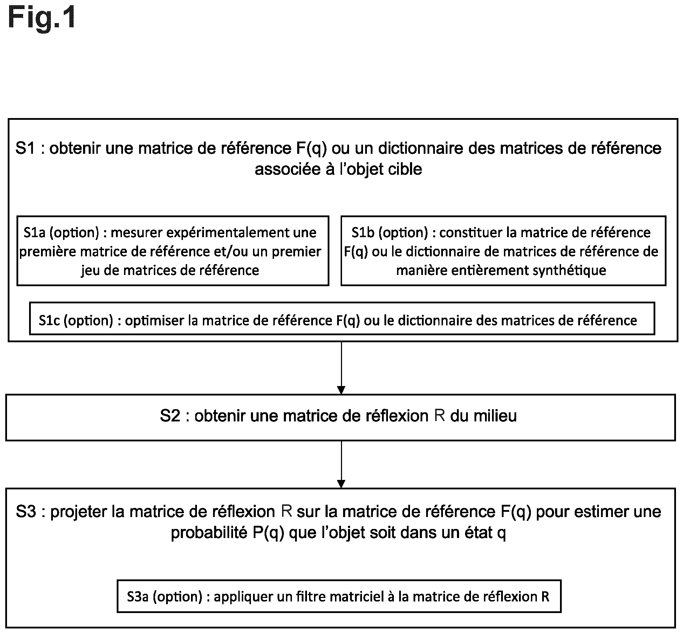

La figure 1 montre schématiquement un procédé pour caractériser un objet cible dans un milieu selon des exemples de la présente divulgation.

L'objet cible peut comprendre par exemple un élément prédéfini du milieu ou région d'intérêt, tel qu'un tissu du milieu et/ou un objet biocompatible ou pas, inséré dans le milieu, tel qu'un dispositif médical, par exemple un marqueur, tel qu'un marqueur utilisé en chirurgie, un dispositif de biopsie, une aiguille ou une sonde.

L'état $q$ de l'objet cible peut comprendre par exemple la position de l'objet dans le milieu, l'orientation de l'objet dans le milieu, la forme et/ou la structure (actuelle) de l'objet, la taille de l'objet, la composition de l'objet, ou des paramètres intensifs locaux du milieu comme la température, la pression ou la concentration d'un composé du milieu environnant la région d'intérêt et/ou une variation de l'état de l'objet.

Selon un exemple de réalisation particulier, l'état $q$ de l'objet peut être mesuré à différents instants et peut ainsi être caractérisé de manière dynamique.

Le procédé selon des exemples de la présente divulgation peut être un procédé d'imagerie ultrasonore. Cependant, d'autres types de procédé d'imagerie ou de scan sont possibles, comme décrit ci-dessous.

Le procédé peut par exemple servir pour reconnaître un objet cible dans un milieu.

Le procédé peut également servir pour générer une cartographie d'un milieu comprenant un objet cible ou susceptible de contenir un objet cible. Selon un exemple de réalisation particulier, la cartographie du milieu consiste en une image du milieu (par exemple obtenue par un procédé prédéfini) et une identification de l'objet cible superposé (par exemple une carte de probabilité de présence de l'objet cible), dans lequel l'identification de l'objet cible est déterminée par un procédé comme décrit ci-dessous. Par exemple, un point à l'emplacement de l'objet cible ou une image (qui peut être une image schématique) de l'objet cible qui se superpose à l'image du milieu.

Le procédé comprend une opération S1 pour obtenir une matrice de référence $F$ ou un dictionnaire des matrices de référence $F(q)$ associées à un état $q$ de l'objet cible.

Les matrices de référence $F$ du dictionnaire peuvent dépendre d'un état de référence de l'objet cible, l'état optionnellement au moins un paramètre parmi : la position, l'orientation, la forme et/ou la structure, la taille, la composition de l'objet ou des paramètres intensifs locaux du milieu comme la température, la pression ou la concentration d'un composé du milieu environnant.

Selon une opération optionnelle S1a, obtenir la matrice de référence $F(q)$ peut comprendre mesurer expérimentalement une première matrice de référence dite matrice de réflexion $R_0(q_0)$ pour un état $q_0$ de l'objet cible, et/ou mesurer expérimentalement un premier jeu de matrices de référence pour un premier jeu d'états $q$.

En outre, l'opération S1a peut comprendre d'obtenir une deuxième matrice de référence pour d'autres valeurs de l'état **q** de l'objet cible en fonction de la première matrice de référence. Par exemple, si l'état **q** est la position **r** de l'objet cible, la deuxième matrice de référence peut être simulée numériquement à partir de la première matrice de réflexion mesurée pour une position **r$_0$** par une translation de l'objet cible à une nouvelle position **r.** Cette translation virtuelle de l'objet peut être réalisée nouvel état **q,** optionnellement dans une base focalisée, la base des transducteurs, ou la base d'ondes planes.

La matrice de réflexion de référence **R$_0$(q)** peut également être simulée numériquement, par exemple en utilisant un modèle de propagation des ondes depuis un réseau de transducteurs jusqu'à l'objet cible et un modèle de diffusion des ondes par l'objet cible.

Selon une opération optionnelle S1b (prise en alternative de l'opération S1a ou en combinaison), l'obtention d'une matrice de référence **F(q)** peut comprendre la constitution d'un dictionnaire de matrices de référence de manière synthétique (c'est à dire obtenu via des simulations et/ou des calculs). Par exemple, une première matrice de réflexion d'un réflecteur virtuel dans un état prédéfini (par exemple un miroir diffusant dont les états sont la position **r$_0$**, l'orientation $\alpha$ et la taille $\ell_c$.) peut être simulée numériquement. Une deuxième matrice de référence **R$_0$(q)** pour un deuxième état **q** de l'objet cible (par exemple à une autre position **r** du miroir) peut être simulée numériquement en fonction de la première matrice de référence. Le dictionnaire de matrices de référence peut également être constitué de manière entièrement synthétique, c'est-à-dire sans mesure expérimentale.

Selon une opération optionnelle S1c, la matrice de référence **F(q)** ou le dictionnaire des matrices de référence peuvent être optimisées en utilisant au moins l'une des méthodes suivantes :

* adaptation du filtre matriciel en fonction de la première matrice de référence afin de rendre le filtre matriciel plus spécifique à l'objet cible recherché par rapport au milieu environnant ;
* optimisation du spectre fréquentiel du dictionnaire pour améliorer le contraste entre l'objet cible et un bruit associé au milieu ;

* construction du dictionnaire à partir d'un estimateur $\mathbf{R}_0^{-1}(\boldsymbol{q})$ de la matrice inverse de la première matrice de référence **R$_0$(q)**.

Dans une opération S2, la matrice de réflexion **R** du milieu peut être obtenue par une méthode d'échographie ultrasonore, par exemple comme décrit dans le dossier FR3114159.

Selon un exemple de réalisation particulier, obtenir la matrice de référence (c'est-à-dire mesurer expérimentalement une première matrice de référence et/ou un premier jeu de matrices de référence) et/ou obtenir la matrice de réflexion comprend :

- une étape de génération d'une série d'ondes incidentes (USin) dans un milieu, au moyen d'un réseau (10) de transducteurs (11), ladite série d'ondes incidentes étant une base d'émission (**i**) ; et
- une étape de génération d'une matrice de réflexion expérimentale **R$_{ui}$**(t) définie entre la base d'émission (**i**) en entrée et une base de réception (**u**) (c'est à dire une base de transducteurs) en sortie ;
- une étape de détermination d'une matrice de réflexion focalisée **R$_{rr}$** =[$R$(**r$_{in}$**, **r$_{out}$**, $\delta$t)] dans une base focalisée, la matrice de réflexion focalisée **R$_{rr}$** comprenant des réponses $R$(**r$_{in}$**, **r$_{out}$**, $\delta$t) du milieu entre un transducteur virtuel d'entrée (TVin) de position spatiale **r$_{in}$**(rin) et un transducteur virtuel de sortie (TVout) de position spatiale **r$_{out}$** (rout), les réponses du transducteur de virtuel de sortie (TVout) étant prises à un instant temporel décalé d'un délai additionnel $\delta$t par rapport à un instant temporel des réponses du transducteur virtuel d'entrée (TVin).

Dans une opération S3, la matrice de réflexion **R** est projetée sur la matrice de référence **F(q)** pour estimer une probabilité $P$(**q**) que l'objet soit dans l'état **q**

Notamment, la projection de la matrice de réflexion **R** peut comprendre projeter la matrice de réflexion **R** sur l'ensemble des matrices de référence **F(q)** du dictionnaire pour estimer une probabilité $P$(**q**) que l'objet soit dans un état **q.**

Selon une opération optionnelle S3a, projeter la matrice de réflexion **R** peut comprendre appliquer un filtre matriciel à la matrice de réflexion **R.** Ce filtre matriciel comprend un produit scalaire normalisé entre la matrice de réflexion **R** et l'ensemble des matrices de référence **F(q)**, ou le filtre matriciel comprend un modèle d'intelligence artificielle construit à partir des matrices de référence. En d'autres termes, le filtre matriciel peut être un corrélateur (produit scalaire normalisé) et/ou une fonction mathématique. Le résultat de ce corrélateur ou de cette fonction mathématique peut être la probabilité estimée. Le modèle d'intelligence artificielle est par exemple un réseau neuronal.

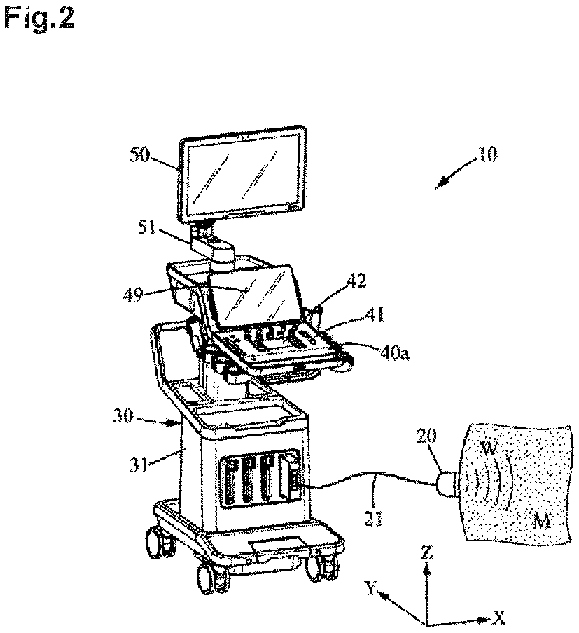

La figure 2 montre un dessin schématique d'un système d'imagerie ultrasonore 10 selon des exemples de la présente divulgation. Il est à noter que le système 10 selon la présente divulgation peut également être un autre type de système qu'un système d'imagerie ultrasonore, comme illustré ci-dessous.

Le système 10 peut avoir des fonctionnalités qui correspondent aux opérations du procédé selon la présente divulgation.

Le système peut être configuré pour caractériser un objet cible dans un milieu. Le système comprend un processeur configuré pour :

obtenir une matrice de référence F($\mathbf{q}$) associée à l'objet cible,
obtenir une matrice de réflexion $\mathbf{R}$ du milieu, et
projeter la matrice de réflexion $\mathbf{R}$ sur la matrice de référence $\mathbf{F(q)}$ pour estimer une probabilité P($\mathbf{q}$) que l'objet soit dans un état $\mathbf{q}$

Le système d'imagerie par ultrasons 10 peut comprendre :

- une sonde 20,
- une unité de traitement 30 pour traiter une image sur la base des signaux reçus par la sonde (par exemple correspondant à l'unité de traitement 11 de la figure 1),
- un panneau de commande 40 relié à l'unité de traitement, ledit panneau de commande comprenant par exemple des boutons 41 et une tablette tactile 42, et
- un écran 50 permettant de visualiser des images.

La sonde 20 peut être connectée à l'unité de traitement 30 par un câble 21 ou par une connexion sans fil, et elle permet d'émettre des ondes ultrasonores W dans un milieu M et de recevoir des ondes ultrasonores W du milieu M, lesdites ondes ultrasonores reçues résultant de réflexions desdites ondes ultrasonores émises sur des particules diffusantes à l'intérieur dudit milieu. La sonde 20 peut être formée ou contenir un réseau de transducteurs comprenant une pluralité de transducteurs, chacun convertissant un signal électrique en une vibration et réciproquement. Un transducteur est par exemple un élément piézoélectrique. Le réseau de transducteurs peut comprendre cent transducteurs ou plus. Le réseau de transducteurs est linéaire ou incurvé et est disposé souvent au travers d'une lentille sur une surface extérieure du milieu M de manière à être couplé au milieu et à vibrer et à émettre ou recevoir des ondes ultrasonores W.
L'unité de traitement 30 peut comprendre des dispositifs de réception pour amplifier et/ou filtrer les signaux reçus de la sonde 20, et des convertisseurs (convertisseurs analogiques-numériques et convertisseurs numériques-analogiques) pour transformer les signaux en données représentant le signal. Les données peuvent être stockées dans une mémoire de l'unité de traitement ou traitées directement pour calculer des données traitées intermédiaires (données de formation de faisceau). L'unité de traitement 30 peut utiliser toute méthode de traitement connue pour traiter l'image sur la base des signaux reçus de la sonde, telle que la formation de faisceau. Les données d'image ultrasonore traitées peuvent être :

- une image simple du milieu (image de réflectivité du milieu obtenue en mode B) généralement en niveaux de gris pour visualiser les organes à l'intérieur du milieu, ou
- une image montrant la vitesse ou l'écoulement dans le milieu (image doppler), par exemple utile pour visualiser les vaisseaux sanguins dans le milieu, ou
- une image montrant une caractéristique mécanique du milieu (élasticité), par exemple utile pour identifier des tumeurs à l'intérieur du milieu (par exemple des données d'image d'élastographie par ondes de cisaillement (ShearWave™ Elastography, ou SWE) comme décrit ci-dessous.

L'écran d'affichage 50 peut être un écran de visualisation de l'image traitée par l'unité de traitement 30.
L'écran d'affichage peut être articulé sur un bras de support 51 pour un meilleur positionnement de l'utilisateur.
Le panneau de commande 40a est par exemple une partie du boîtier du système 31, ladite partie comprenant un boîtier de panneau ayant une surface sensiblement plane inclinée vers l'utilisateur pour être manipulée par une main dudit utilisateur.
La sonde à ultrasons 20 peut comprendre par exemple un ou plusieurs éléments transducteurs non représentés sur la figure 1), chacun étant configuré pour convertir un signal électrique reçu du système 10 en ondes ultrasonores. Les éléments transducteurs peuvent être configurés pour transmettre (a) les ondes dans le milieu et/ou pour recevoir (b) une pluralité de signaux ultrasonores du milieu, éventuellement en réponse à la transmission (a).
Le système 10 peut être un système médical, par exemple un système à ultrasons. Par exemple, le système peut être associé à une sonde à ultrasons 20, afin de collecter des données ultrasonores d'un milieu, par exemple composé de tissus vivants et/ou en particulier de tissus humains ou animaux.
Toutefois, le système 10 peut être tout type de système électronique. Par exemple, le système peut également être un autre type de système médical qu'un système d'imagerie par ultrasons. En conséquence, la sonde 20 peut être tout type de dispositif d'imagerie ou de capteur, utilisant d'autres ondes que les ondes ultrasonores (par exemple, des ondes ayant une longueur d'onde différente de la longueur d'onde des ultrasons et/ou des ondes n'étant pas des ondes sonores).

Des exemples de systèmes d'imagerie médicale comprennent un système d'imagerie par ultrasons, un système d'imagerie par rayons X (en particulier pour la mammographie) et un système IRM (Imagerie par résonance magnétique).

Selon d'autres exemples, le système 10 peut comprendre au moins une unité de traitement (ou processeur) et en plus une mémoire (non représentée). Dans des exemples, l'unité de traitement et de mémoire peut être incorporée dans le système ou peut être un ordinateur ou un dispositif informatique lié de manière communicative à celui-ci. Selon la configuration exacte et le type de dispositif informatique, la mémoire (qui stocke les instructions permettant d'évaluer les données ultrasonores ou d'exécuter les procédés décrits dans le présent document) peut être volatile (telle que la RAM), non volatile (telle que la RAM, la mémoire flash, etc.) ou une combinaison des deux. En outre, le système 10 peut également comprendre des dispositifs de stockage (amovibles et/ou non amovibles), y compris, mais sans s'y limiter, des disques magnétiques ou optiques ou des bandes.

Le système 10 peut être un ordinateur unique fonctionnant dans un environnement en réseau utilisant des connexions logiques avec un ou plusieurs ordinateurs distants. L'ordinateur distant peut être un ordinateur personnel, un serveur, un routeur, un PC de réseau, un dispositif homologue ou un autre noeud de réseau commun, et comprend généralement plusieurs ou tous les éléments décrits ci-dessus ainsi que d'autres non mentionnés. Les connexions logiques peuvent inclure toute méthode supportée par les supports de communication disponibles. De tels environnements de mise en réseau sont courants dans les bureaux, les réseaux informatiques d'entreprise, les intranets et l'Internet.

En outre, l'unité de traitement peut être configurée pour traiter des données et/ou envoyer des données à un dispositif externe, comme par exemple, un dispositif d'affichage, un serveur, un ordinateur sur lequel un algorithme d'intelligence artificielle (IA) est exécuté, une station de travail dédiée, ou tout autre dispositif externe.

La description suivante résume le contexte technique et scientifique de la présente divulgation et illustre la méthode de la présente divulgation à l'aide de plusieurs exemples et expériences concrets. Ces exemples de réalisation ne limitent pas la présente divulgation, mais servent principalement à une meilleure compréhension, y compris des avantages de la présente divulgation.

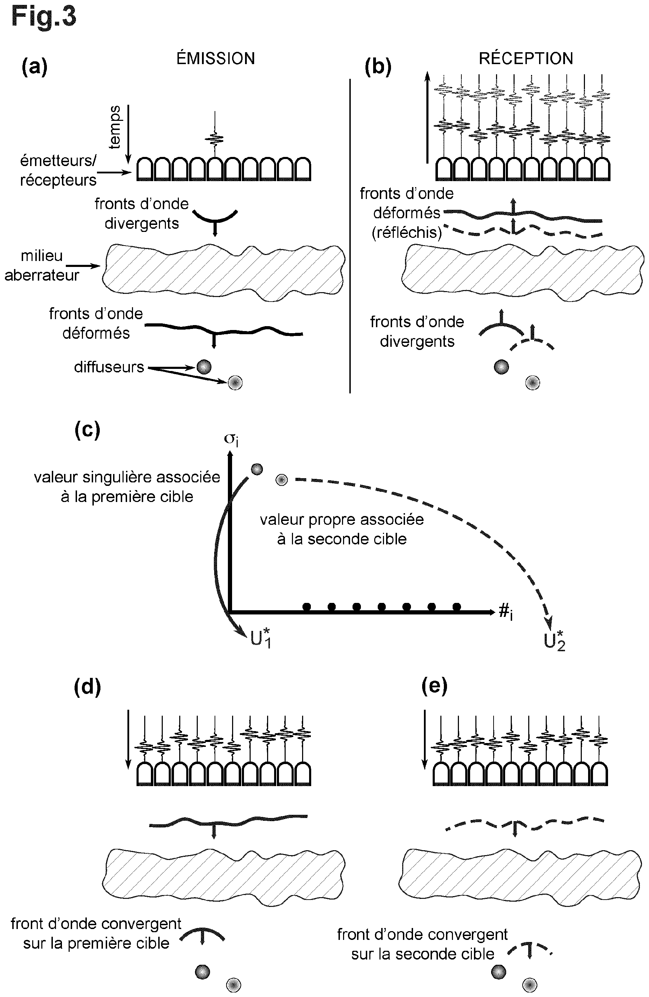

La figure 3 montre schématiquement le principe d'une méthode pour décomposition de l'opérateur retournement temporel selon un exemple.

La partie (a) de la figure 3 montre schématiquement une onde incidente, qui est émise par un des transducteurs et se propage à travers le milieu jusqu'à la cible (schéma de gauche). La partie (b) de la figure 3 montre schématiquement que cette onde est ensuite réfléchie par les diffuseurs présents dans le milieu étudié, se propage de nouveau à travers le milieu. Les échos rétrodiffusés sont alors enregistrés par le réseau de transducteurs. Cette opération est répétée pour chaque élément du réseau agissant comme source d'émission d'ondes. L'ensemble des réponses impulsionnelles entre les transducteurs du réseau est stocké dans la matrice de réflexion $\mathbf{R}$ ainsi acquise dans la base canonique. La partie (c) de la figure 3 montre schématiquement le spectre des valeurs singulières de la matrice $\mathbf{R}$. Les parties (d) et (e) de la figure 3 montrent schématiquement, que chaque vecteur propre $\mathbf{U}_i$ associé à une valeur singulière significative de $\mathbf{R}$ correspond au front d'onde, qui est à ré-émettre depuis la sonde pour focaliser sélectivement sur le diffuseur associé. L'onde transmise est alors focalisée de manière optimale sur chaque cible à travers le milieu.

Généralement, en échographie, un réseau de transducteurs piézoélectriques est placé en vis-à-vis du milieu que l'on souhaite imager (cf. la partie (a) de la figure 3). Ces éléments peuvent émettre/recevoir des ondes ultrasonores de manière indépendante sur une large bande spectrale. A partir d'une série d'insonifications du milieu par tout ou partie de ce réseau, les échos rétrodiffusés par les hétérogénéités du milieu sont enregistrés soit par ces mêmes transducteurs soit par d'autres transducteurs dédiés. Les signaux ainsi enregistrés sont ensuite stockés dans une matrice de réponse $\mathbf{R}$ (c'est-à-dire par exemple une matrice de réflexion). Cette matrice peut être acquise pour différents types d'ondes incidentes.

Une séquence d'acquisition simple consiste à émettre chaque onde incidente avec un élément à la fois (cf. la partie (a) de la figure 3) et, pour chaque émission, à enregistrer le champ réfléchi par le milieu sur tous les éléments du réseau (cf. la partie (b) de la figure 3). Cette base canonique est utilisée dans le domaine du contrôle non destructif, où elle est appelée "full matrix capture". Une matrice acquise de cette manière peut s'écrire mathématiquement comme $\mathbf{R}_{uu}(t)=[R(\mathbf{u}_{out},\mathbf{u}_{in},t)]$, où $\mathbf{u}$ est la position des éléments le long du réseau, les indices 'in' et 'out' indiquant respectivement l'émission et la réception. La matrice de réponse peut également être acquise en utilisant la formation de faisceaux (émission et/ou réception avec tous les éléments de concert avec des délais appropriés entre chaque élément) afin de synthétiser, par exemple, des faisceaux focalisés comme réalisé en imagerie B-mode ou des ondes planes pour augmenter la cadence d'image Dans ce dernier cas, pour chaque onde plane dont la direction est donnée par le vecteur unitaire $\hat{\theta}_{in}$, le champ réfléchi enregistré par les transducteurs est stocké dans une matrice de réponse notée $\mathbf{R}_{u\theta}(t) = [R(\mathbf{u}_{out}, \hat{\theta}_{in}, t)]$. Il est considéré dans la suite, des données ultrasonores acquises dans la base des ondes planes à l'émission et dans la base des transducteurs à la réception. Mais la présente divulgation est générale et peut s'appliquer à n'importe quelle base d'acquisition.

A partir de la matrice de réponses, une image ultrasonore peut être formée en additionnant de manière cohérente les échos enregistrés provenant de chaque point focal $\mathbf{r}_{out}$, lequel agit alors comme un détecteur virtuel à l'intérieur du milieu. En pratique, des délais appropriés sont appliqués aux signaux enregistrés avant leur sommation. Les images obtenues pour chaque onde incidente sont ensuite additionnées de manière cohérente et donnent lieu à une image finale dont le contraste est amélioré. Cette dernière opération génère a posteriori une focalisation synthétique (c'est-à-dire une source virtuelle) sur chaque point focal $r_{in} = r_{out}$. L'image composée est ainsi équivalente à une image confocale qui serait obtenue en focalisant les ondes sur le même point en mode émission et en mode réception.

L'imagerie matricielle consiste à découpler les points de focalisation $\mathbf{r}_{in}$ et $\mathbf{r}_{out}$. Une matrice de réflexion focalisée, $\mathbf{R_{rr}}(\delta t)=[R(\mathbf{r}_{in}, \mathbf{r}_{out}, \delta t)]$, peut ainsi être synthétisée par sommation et application de temps de retards aux signaux IQ de la matrice de réponse enregistrée. Pour la matrice de réflexion enregistrée dans la base des ondes planes, l'opération de double focalisation s'exprime mathématiquement de la manière suivante :

[Math 1]

$$R(\mathbf{r}_{in}, \mathbf{r}_{out}, \delta t) = \sum_{\widehat{\boldsymbol{\theta}}_{in}, \mathbf{u}_{out}} R\left(\widehat{\boldsymbol{\theta}}_{in}, \mathbf{u}_{out}, \delta t + \tau_{in}\left(\widehat{\boldsymbol{\theta}}_{in}, \mathbf{r}_{in}\right) + \tau_{out}(\mathbf{u}_{out}, \mathbf{r}_{out})\right)$$

avec $\tau_{in}$ et $\tau_{out}$ les lois de retard spatio-temporelles à appliquer en entrée et sortie de la matrice $\mathbf{R_{rr}}(\delta t)$. Ces lois de retard dépendent de notre connaissance *a priori* de la distribution de vitesse du son $c(\mathbf{r})$ dans le milieu. Pour un modèle de vitesse homogène ($c(\mathbf{r})=c_0$), $\tau_{in}$ et $\tau_{out}$ s'expriment de la manière suivante :

[Math 2]

$$\tau_{in}\left(\widehat{\boldsymbol{\theta}}_{in}, \mathbf{r}_{in}\right) = \widehat{\boldsymbol{\theta}}_{in} \cdot \mathbf{r}_{in}/c_0$$

et

$$\tau_{out}(\mathbf{u}_{out}, \mathbf{r}_{out}) = \|\mathbf{u}_{out} - \mathbf{r}_{out}\|/c_0$$

avec $(X_{in}, Z_{in})$ et $(X_{out}, Z_{out})$, les coordonnées des points de focalisation $r_{in}$ et $r_{out}$.

Cette projection de la matrice $\mathbf{R}(t)$ dans une base focalisée peut également être réalisée via des produits matriciels dans le domaine fréquentiel. Pour cela, une première étape consiste à réaliser une transformée de Fourier temporelle des signaux mesurés. Une matrice de réflexion monochromatique $\tilde{\mathbf{R}}(f)$ est obtenue à chaque fréquence $f$. Pour une acquisition initiale de $\mathbf{R}$ dans la base des ondes planes, la projection de $\mathbf{R_{u\theta}}(f)$ dans la base focalisée s'effectue via le produit matriciel suivant :

[Math 3]

$$\tilde{\mathbf{R}}_{\mathbf{rr}}(f) = \mathbf{G}_{\mathbf{ur}}^{\dagger}(f) \times \tilde{\mathbf{R}}_{\mathbf{u\theta}}(\omega) \times \mathbf{P}_{\boldsymbol{\theta r}}^{*}(f)$$

dans lequel les matrices $\mathbf{P}_{\boldsymbol{\theta r}}(f) = [P(\hat{\theta}, \mathbf{r}, f)]$ et $\mathbf{G}_{\mathbf{ur}}(f) = [G(\mathbf{u}, \mathbf{r}, f)]$ sont les matrices de passage de la base focalisée à, respectivement, la base des ondes planes et celle des transducteurs. $\mathbf{P}_{\boldsymbol{\theta r}}$ et $\mathbf{G}_{\mathbf{ur}}$ dépendent de notre connaissance *a priori* de la distribution de vitesse du son $c(\mathbf{r})$ dans le milieu. Pour un modèle de vitesse homogène, les coefficients de $\mathbf{P}_{\boldsymbol{\theta r}}$ sont donnés par :

[Math 4]

$$P\left(\widehat{\boldsymbol{\theta}}, \mathbf{r}, f\right) = \exp\left(ik\mathbf{r} \cdot \widehat{\boldsymbol{\theta}}\right)$$

avec $k=2\pi f/c_0$, le nombre d'onde. Les coefficients de $\mathbf{G}_{\mathbf{ur}}$ correspondent aux fonctions de Green 2D ou 3D de l'équation des ondes en milieu homogène :

[Math 5],

$$G^{2D}(\mathbf{u}, \mathbf{r}) = G_{2D}(\mathbf{u}, \mathbf{r}, f) = -\frac{i}{4}\mathcal{H}_0(k|\mathbf{u} - \mathbf{r}|)$$

avec $\mathcal{H}_0$ la fonction de Hankel du 1er ordre dont l'expression asymptotique est la suivante :

$$\mathcal{H}_0(2\pi f|\mathbf{u} - \mathbf{r}|/c_0) \approx e^{3i\pi/4}\sqrt{\frac{2}{\pi}}\frac{e^{-jk_0|\mathbf{u}-\mathbf{r}|}}{\sqrt{k_0|\mathbf{u}-\mathbf{r}|}}$$

[Math 6]

$$G_{3D}(\mathbf{u}, \mathbf{r}, f) = \frac{\exp(-ik_0|\mathbf{u} - \mathbf{r}|)}{4\pi|\mathbf{u} - \mathbf{r}|}$$

Une transformée de Fourier inverse de $\tilde{\mathbf{R}}_{\mathbf{rr}}(f)$ peut ensuite être réalisée pour obtenir la matrice de réflexion focalisée $\mathbf{R}_{\mathbf{rr}}(\delta t)$ dans le domaine temporel.

La présente divulgation concerne l'utilisation de la matrice $\mathbf{R}_{\mathbf{rr}}(\delta t)$ à des fins de détection et de localisation de cibles (c'est-à-dire des objets cibles) dans des milieux hétérogènes.

**Détection de cibles enfouies dans les milieux diffusants** : **La méthode pour décomposition de l'opérateur retournement temporel**

La matrice de réponse **R** peut être exploitée pour la détection de cibles (c'est-à-dire des objets cibles). Physiquement, cette méthode permet de focaliser sélectivement par retournement temporel itératif sur chaque diffuseur d'un milieu multi-cibles. Mathématiquement, elle consiste en une décomposition en valeurs propres de l'opérateur de retournement temporel $\tilde{\mathbf{R}}\tilde{\mathbf{R}}^\dagger$ dans le domaine fréquentiel. En régime de diffusion simple et pour des cibles ponctuelles, chaque espace propre de $\tilde{\mathbf{R}}\tilde{\mathbf{R}}^\dagger$ est associé à un diffuseur. Une décomposition en valeurs singulières (SVD) de $\tilde{\mathbf{R}}$ est réalisée pour calculer les espaces propres de $\tilde{\mathbf{R}}\tilde{\mathbf{R}}^\dagger$ :

[Math 7]

$$\tilde{\mathbf{R}} = \mathbf{U} \times \boldsymbol{\Sigma} \times \mathbf{V}^\dagger$$

où $\Sigma$ est une matrice (diagonale) dont les coefficients diagonaux $\sigma_i$ sont les valeurs singulières,

**V** et **U** sont des matrices unitaires dont les vecteurs colonnes,

$\mathbf{V}_i$ et $\mathbf{U}_i$ sont les vecteurs singuliers en entrée et sortie de $\tilde{\mathbf{R}}$.

En régime de diffusion simple, chaque espace propre est associé à une cible. La valeur singulière $\sigma_i$ correspondante est proportionnelle à la réflectivité de la cible. Si la matrice $\tilde{\mathbf{R}}_{\mathbf{u\theta}}$ est considérée, le conjugué en phase du vecteur propre, $\mathbf{U}_{\mathbf{u},i}$, correspond au front d'onde à émettre depuis la sonde pour focaliser sur le diffuseur associé (cf. les parties (d) et (e) de la figure 3). Une image de la cible, $I_i^{(D)}$, est obtenue en repropageant numériquement le vecteur propre associé :

[Math 8]

$$I_i^{(D)} = \mathbf{G}_{\mathbf{ur}}^\dagger \times \mathbf{U}_{\mathbf{u},i}$$

Si la méthode pour décomposition de l'opérateur retournement temporel est appliquée à la matrice $\mathbf{R}_{\mathbf{rr}}$ focalisée, le vecteur propre associé $\mathbf{U}_{\mathbf{r},i}$ fournit l'image du diffuseur. Par rapport à une image échographique conventionnelle, l'image obtenue par cette méthode souffre d'une pauvre résolution axiale. En effet l'analyse pour décomposition de l'opérateur retournement temporel est monochromatique. La résolution axiale est donc donnée par la profondeur de champ de la sonde et non par la résolution temporelle des signaux ultrasonores.

La force de la méthode pour décomposition de l'opérateur retournement temporel réside dans le fait que l'association biunivoque entre espaces propres et diffuseurs reste valable en présence d'aberrations. Toutefois, la détection de cible n'est possible que si celle-ci présente un pouvoir diffusant plus important que le speckle ultrasonore ambiant. De plus, en régime de diffusion multiple, la matrice de réflexion doit être au préalable filtrée pour éliminer une large part de la diffusion multiple avant de pouvoir lui appliquer la méthode pour décomposition de l'opérateur retournement temporel. Dans tous les cas, la cible pourra être détectée si la profondeur de pénétration reste limitée à un libre parcours moyen de diffusion $\ell_s$ (distance moyenne entre deux évènements de diffusion successifs).

Enfin, la relation biunivoque entre un espace propre et un diffuseur n'est vérifiée que si la taille de la cible est plus petite qu'une cellule de résolution du système d'imagerie. Dans le cas contraire, le rang de la matrice $\tilde{R}$ est de l'ordre du nombre Q de cellules de résolution contenu dans l'objet. L'image de la cible est obtenue en combinant les Q premiers espaces propres de $\tilde{R}\tilde{R}^{\dagger}$.

## Localisation et caractérisation d'objets : Le tenseur de polarisation généralisé

Pour les ondes électromagnétiques, il a été développé une méthode mathématique reposant sur la matrice de réponse monochromatique et le tenseur de polarisation généralisé (GPT) de l'objet sondé (ici, une inhomogénéité diélectrique). Lorsqu'un réseau d'antennes entoure l'objet, le GPT de la cible peut être obtenu avec précision à partir de la matrice $\tilde{R}\tilde{R}^{\dagger}$ en résolvant un système d'équations linéaire. Sur cette base, on obtient un algorithme rapide qui identifie une cible à partir d'un dictionnaire de données GPTs précalculées. La localisation et la forme de l'objet sont caractérisées à partir d'un élément du dictionnaire après une certaine rotation, mise à l'échelle et translation. Cette procédure de correspondance de dictionnaire opère directement dans les données GPTs.

La présente divulgation est basée sur le concept de détection et de caractérisation de cibles à partir d'un dictionnaire de données précalculées ou pré-enregistrées mais directement sur la matrice de réflexion large bande. Ainsi, il n'y a pas de système d'équation à résoudre, ce qui rend la méthode beaucoup plus simple et robuste que l'approche GPT. En outre, elle ne requiert pas un réseau de transducteurs entourant le milieu étudié. Enfin, contrairement à la méthode pour décomposition de l'opérateur retournement temporel ou l'approche GPT, l'analyse multispectrale permet de retrouver avantageusement une résolution axiale dictée par la bande passante de la sonde, et non par sa profondeur de champ. En outre, cela permet une exploitation optimale de la réponse temporelle (ou spectrale) de la cible pour sa détection dans des milieux complexes où sa présence est généralement cachée par un fort bruit de multi-diffusions.

## Le procédé selon des exemples de la présente divulgation

La figure 3 montre schématiquement un diagramme résumant les différentes étapes de la méthode de projection matricielle à partir d'une mesure de calibration d'une matrice de réflexion $R_0(r_0)$ selon des exemples de la présente divulgation.

## Obtenir un dictionnaire des matrices de référence

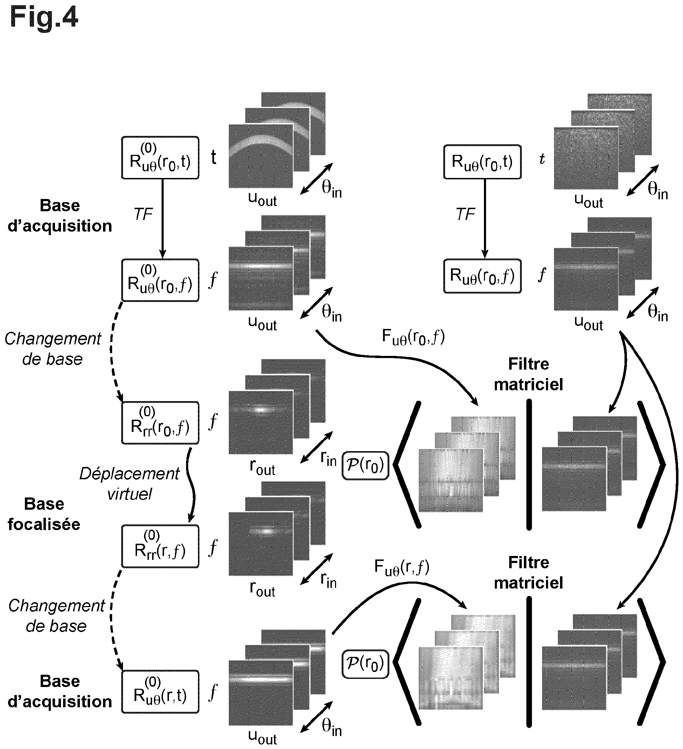

La première étape consiste à constituer un dictionnaire de matrices de référence $F(r, \{q_i\})$ dépendant de la position $r$ de la cible que l'on cherche à détecter et d'un ensemble de paramètres $q_i$ qui désignent par exemple sa taille, sa forme, son orientation, sa composition, des paramètres intensifs locaux du milieu environnant, tels que comme la température, la pression, la température, la composition, des propriétés mécaniques et/ou la concentration d'un composé du milieu environnant, *etc.* En pratique, ce dictionnaire peut être constitué à l'aide d'une étape de calibration consistant à mesurer expérimentalement la matrice de réflexion $R_0(r, \{q_i\})$ associée à la cible pour un ensemble de positions $r$ de celle-ci et un ensemble de valeurs des paramètres $q_i$. Il peut être également généré numériquement à l'aide d'un modèle de propagation des ondes depuis la sonde jusqu'à l'objet et un modèle de diffusion des ondes par ce dernier. Enfin, les deux approches peuvent être combinées en mesurant expérimentalement la matrice de réflexion $R_0(r_0, q)$ pour une position $r_0$ de la cible puis en en déduisant la valeur de $F$ pour chaque position $r$ dans le champ de vision (cf. La figure 4). La même méthode ou un modèle d'intelligence artificielle peut être utilisée pour déterminer la dépendance de $F(r, \{q_i\})$ vis à vis d'autres paramètres $q_i$ caractérisant la cible.

Une fois ce dictionnaire de matrices de référence constitué, la seconde étape consiste à acquérir la matrice de réflexion $R$ du milieu que l'on cherche à caractériser. Le filtre matriciel consiste ensuite à réaliser un produit scalaire normalisé entre la matrice mesurée et l'ensemble des matrices de référence $F(r, q)$ afin d'estimer la probabilité $P(r, q)$ que l'objet soit à la position $r$ et dans l'état $q$. Ce produit scalaire peut être réalisé dans le domaine temporel,

[Math 9]

$$P(\mathbf{r}, \mathbf{q}) = \frac{\left| \int dt \, \mathrm{Tr}\{\mathbf{R}(t)\mathbf{F}^{\dagger}(\mathbf{r}, \mathbf{q}, t)\} \right|}{[\int dt \, \|\mathbf{R}(t)\|_2 \times \int dt \, \|\mathbf{F}(\mathbf{r}, \mathbf{q}, t)\|_2]^{1/2}}$$

ou, dans le domaine fréquentiel,

[Math 10]

$$P(\mathbf{r}, \mathbf{q}) = \frac{\left| \int df \, \mathrm{Tr}\{\mathbf{R}(f)\mathbf{F}^{\dagger}(\mathbf{r}, \mathbf{q}, f)\} \right|}{[\int dt \, \|\mathbf{R}(f)\|_2 \times \int dt \, \|\mathbf{F}(\mathbf{r}, \mathbf{q}, f)\|_2]^{1/2}}$$

avec $\mathrm{Tr}\{\mathbf{A}\}$, la trace de la matrice $\mathbf{A}$, et $\|\mathbf{A}\|_2 = \mathrm{Tr}\{\mathbf{AA}^{\dagger}\}$.

Si le projecteur $\mathbf{F}$ (c'est-à-dire par exemple. le dictionnaire des matrices de référence) est pris égal à la matrice $\mathbf{R_0}$, les équations [Math 9] et [Math 10] correspondent à un filtre adapté et optimal pour l'objet recherché de la matrice $\mathbf{R}$. Le résultat $P(\mathbf{r}, \mathbf{q})$ de la méthode de projection matricielle correspond alors à la somme cohérente des coefficients de corrélation entre les éléments des matrices $\mathbf{R_0}$ et $\mathbf{R}$ intégrée sur la bande passante des signaux ultrasonores. L'opérateur $\mathbf{RR_0^{\dagger}}$ diffère de l'opérateur de retournement temporel $\mathbf{RR}^{\dagger}$ est considéré par la méthode pour décomposition de l'opérateur retournement temporel. Il est en réflexion du 'Scattering Invariant Mode' (SIM) operator $\mathbf{TT_0^{\dagger}}$ introduit par la référence Pai et.al. dans une configuration en transmission, cf. Pai, P., Bosch, J., Kühmayer, M. et al. Scattering invariant modes of light in complex media. Nat. Photonics 15, 431-434 (2021). La matrice $\mathbf{T}$ représente la matrice de réponse entre deux réseaux de transducteur placés de part et d'autre du milieu et la matrice $\mathbf{T_0}$ est la matrice de référence qui serait obtenue si le milieu était homogène.

Suivant la configuration étudiée et la qualité de la mesure de calibration, le projecteur $\mathbf{F}$ peut prendre des formes plus élaborées :

(*i*) en filtrant la matrice $\mathbf{R_0}$ afin de rendre le filtre matriciel plus spécifique à la cible recherchée par rapport au milieu environnant ;
(*ii*) en blanchissant son spectre fréquentiel ;
(*iii*) en modifiant la nature du filtre matriciel. Par exemple, le projecteur $\mathbf{F}$ peut être construit à partir d'un estimateur $\widehat{\mathbf{R}}_0^{-1}$ de la matrice inverse de $\mathbf{R_0}$.

En prenant $\mathbf{F}^{\dagger} = \widehat{\mathbf{R}}_0^{-1}$, les équations [Math 9] et [Math 10] correspondent à une opération de filtrage inverse de la matrice $\mathbf{R}$. A titre d'illustration, plusieurs exemples de matrices $\mathbf{F}$ seront considérés par la suite.

Ci-dessus la méthode de projection matricielle a été définie mathématiquement (Eqs. [Math 9] et [Math 10]). Dans la suite, ses avantages seront illustrés dans le contexte de deux exemples pour la détection et localisation de cibles ainsi que pour une imagerie ultrasonore quantitative des tissus biologiques.

**Détection et localisation de cibles enfouies dans un milieu diffusant**

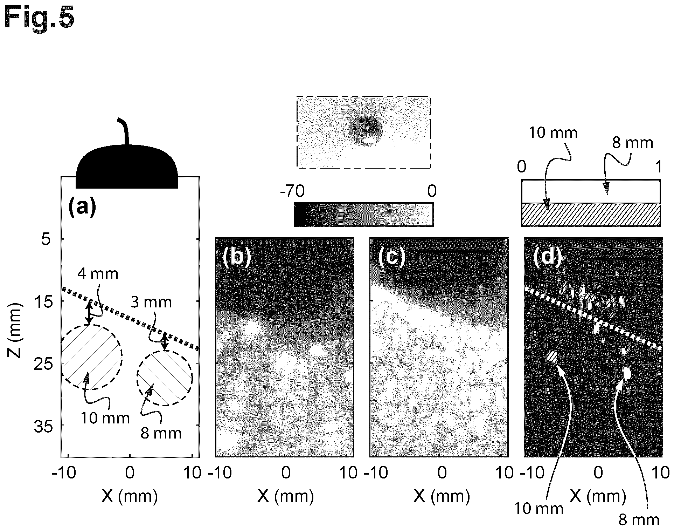

La figure 5 montre schématiquement une preuve de concept selon une première expérimentation comprenant une imagerie matricielle de sphères d'acier dans des milieux granulaires fortement diffusants selon des exemples de la présente divulgation. La partie (a) de la figure 5 montre schématiquement un dispositif expérimental : Une sonde matricielle mesure la matrice de réflexion $\mathbf{R}$ associée au milieu d'intérêt, deux sphères en acier enfouies dans un milieu granulaire particulièrement diffusant. Les parties (b), (c) de la figure 5 montrent schématiquement une image ultrasonore des deux sphères, respectivement, en absence et en présence du milieu granulaire. La partie (d) de la figure 5 montre schématiquement une carte de vraisemblance de position pour chaque intrus résultant du filtre matriciel adapté.

Pour la première expérience, le dispositif expérimental consiste en une sonde matricielle 2D placée devant une suspension granulaire fortement diffusante constituée d'un empilement aléatoire de billes de verre de $315 \, \mu m$ de diamètre immergées dans l'eau. Un filtre en fréquence a été appliqué pour réduire la bande passante sur un domaine où les propriétés de transport de l'onde peuvent être considérées comme relativement constantes. A partir de mesures de transmission de l'impulsion cohérente, on estime la vitesse de phase longitudinale $c_\varphi \sim$1.6 mm/$\mu$s et le libre parcours de diffusion $\ell_s$ est de l'ordre de 1 mm dans la bande passante étudiée. $\ell_s$ correspond à la distance moyenne entre deux évènements de diffusion successifs subis par l'onde. Les objets qu'on souhaite imager sont deux sphères en acier de 8 mm et 10 mm de diamètre dont le centre est enterré à z$\sim$7 $\ell_s$ et 9$\ell_s$ sous la surface du milieu (cf. la partie (a) de la figure 5). Ces conditions d'imagerie sont donc particulièrement désavantageuses car l'onde simplement diffusée est atténuée exponentiellement par la diffusion du milieu granulaire d'un facteur exp (-z/$\ell_s$). La partie (c) de la figure 5 montre l'image confocale de l'ensemble du milieu. La présence des deux cibles n'est pas révélée en raison d'un fond de diffusion multiple largement prédominant. De manière très avantageuse, la méthode de projection matricielle décrite plus haut permet de construire une carte de vraisemblance pour chaque cible (cf. la partie (a) de la figure 5) et leurs positions exactes sont retrouvées sans ambiguïté.

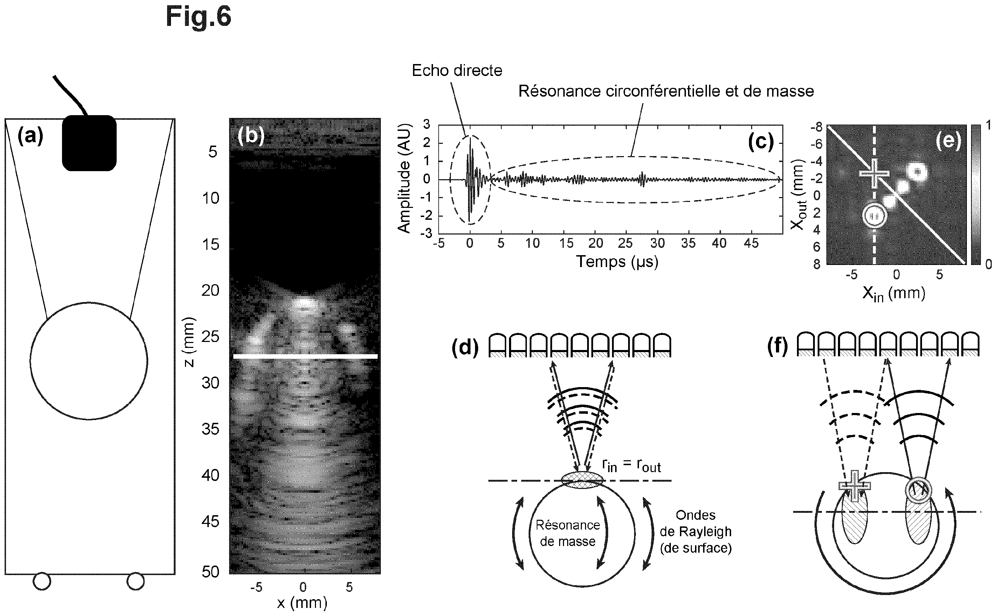

La figure 6 montre schématiquement un dispositif et méthode pour obtenir une matrice de référence $\mathbf{R_0}$ mesurée expérimentalement dans le contexte de la première expérience selon des exemples de la présente divulgation. Le dispositif et méthode peuvent servir pour la détection et localisation de cibles enfouies dans un milieu diffusant selon la présente divulgation. Cette expérience utilise une matrice de référence $\mathbf{R_0}$ mesurée pour une position de la sphère en acier ($\phi$ = 8 mm). La partie (a) de la figure 6 montre schématiquement un dispositif expérimental utilisé pour l'enregistrement de $\mathbf{R_0}$. La partie (b) de la figure 6 montre schématiquement une image réalisée en mode-B (B- « brightness » scan de l'image ultrasonore). La partie (c) de la figure 6 montre schématiquement la dépendance temporelle du signal confocal au niveau de la calotte de la sphère. La partie (d) de la figure 6 montre schématiquement la résonance des ondes de volume et de surface expliquant la longue queue temporelle du signal visible dans les parties (b) et (c). La partie (e) de la figure 6 montre schématiquement la matrice $\mathbf{R_0}$ dans la base focalisée à la profondeur z=27 mm de l'image ultrasonore [ligne horizontale sur la partie (b)]. La partie (f) de la figure 6 montre schématiquement des ondes de surface expliquant le signal hors-diagonal dans la partie (e).

Ce résultat avantageux décrit dans le contexte de la figure 5 a été obtenu en mesurant dans une première étape de calibration la matrice de réflexion associée à chacune des billes pour une position donnée (cf. la partie (a) de la figure 6). Ces matrices peuvent donc former un dictionnaire des matrices de référence. Grâce à un processus décrit ci-dessous, on peut virtuellement déplacer l'objet dans le champ de vision et déterminer l'évolution spatiale de $\mathbf{R_0}(\mathbf{r}, \phi)$. La signature spatio-temporelle complexe des billes est illustrée par la figure 6 qui illustre le cas de la sphère en acier de 8 mm dans l'eau. Son signal confocal présente un long temps de réverbération (cf. la partie (c) de la figure 6) dû aux résonances de Mie (ondes volumiques) (cf. la partie (d) de la figure 6) et de Rayleigh (surface) (cf. la partie (f) de la figure 6) que peut générer une telle bille. La matrice de réflexion présente également une signature spatiale complexe mise en évidence par une forte énergie hors diagonale dans la base focalisée (cf. la partie (e) de la figure 6). Notamment, pour mettre en exergue la spécificité de la signature spatio-temporelle du cible par rapport à la signature des objets environnants, un filtre temporel peut être appliqué au matrice de réflexion pour ne garder que les échos enregistrés aux temps longs, correspondant aux ondes de surface et de volume se propageant sur et dans le cible.

Cette complexité de la signature spatio-temporelle de chaque bille peut être quantifiée par les nombres de degrés de liberté spatiaux et temporels, $N_s$ et $N_t$, contenus dans cette signature. $N_s$ est égal au rang effectif de la matrice $\mathbf{R_0}$, soit approximativement le nombre de cellules de résolution transverses occupées par la cible. $N_t$ est égal au produit du temps de réverbération de la cible par la largeur de bande de fréquence. Dans le cas présent, la taille étendue des cibles ($N_s \sim$20) associée à leur longue queue temporelle ($N_t \sim$20)) donne lieu à un nombre élevé de degrés de liberté spatio-temporels. En comprimant en un seul point le signal associé à chaque cible, la projection matricielle développée ici exalte le ratio diffusion simple-sur-multiple d'un facteur M= $N_s \times N_t \sim$ 400. Ce gain considérable permet de compenser l'atténuation exponentielle des ondes simplement diffusées dans le milieu granulaire [[exp(-z/$\ell_s$) $\sim$2 - 5 $\times$ 10$^{-2}$]]. Une détection de chaque cible et leur localisation sont obtenues comme si le milieu granulaire avait été rendu soudainement homogène (cf. la partie (d) de la figure 5).

## Détection et localisation d'un marqueur de lésion noyé dans le speckle ultrasonore

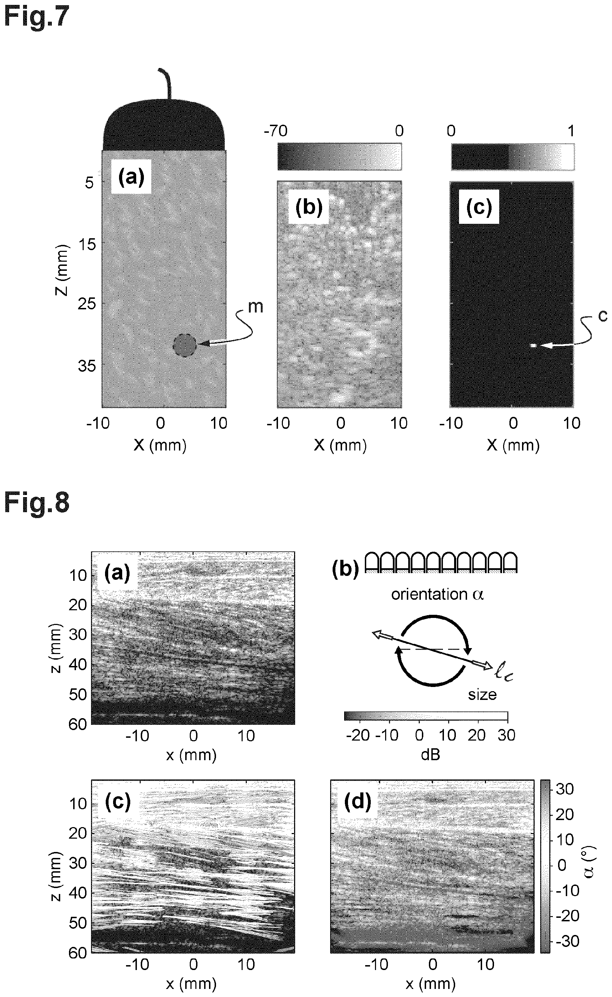

La figure 7 illustre schématiquement une méthode de détection et localisation d'un marqueur de lésion dans le speckle ultrasonore selon une deuxième expérimentation menée selon des exemples de la présente divulgation. La partie (a) de la figure 7 montre schématiquement un marqueur m de lésion sphérique 3D dans un dispositif expérimental. La partie (b) de la figure 7 montre schématiquement 'image ultrasonore obtenue en mode B. La partie (c) de la figure 7 montre schématiquement une carte de vraisemblance de position c pour le marqueur résultant du filtre matriciel adapté.

Une seconde expérience a été réalisée consistant à détecter et localiser un marqueur de lésion utilisé pour le marquage des sites de biopsie et des lésions suspectes dans les tissus mammaires. Pour cette expérience, le marqueur est positionné dans une mousse immergée dans l'eau. Cette mousse génère un speckle ultrasonore typique, par exemple des tissus mammaires. La présence du marqueur est difficilement décelable sur l'image ultrasonore conventionnelle acquise avec la sonde 2D (cf. la figure 7). En revanche, la présente divulgation mise en oeuvre via projecteur matriciel exploite la signature matricielle du marqueur pour le détecter avec un excellent contraste et le localiser avec une précision bien inférieure à la limite de diffraction.

**Imagerie de l'anisotropie des tissus fibreux**

La figure 8 montre schématiquement un exemple d'imagerie matricielle d'un mollet, dans lequel le dictionnaire de matrices de référence est constitué de manière entièrement synthétique selon la présente divulgation.

La partie (a) de la figure 8 montre schématiquement une image échographique du mollet en dB ($c_0$ = 1580 m/s). La partie (b) de la figure 8 montre schématiquement une configuration expérimentale simulée numériquement (modèle de vitesse du son homogène $c_0$) pour établir un dictionnaire de matrices $\mathbf{R_0}(\mathbf{r}, \{\alpha, \ell_c\})$ associées à un miroir d'orientation $\alpha$ et de taille $\ell_c$. La partie (c) de la figure 8 montre schématiquement l'orientation et la taille locale des fibres, $\alpha_c^{(opt)}(\mathbf{r})$ et $\ell_c^{(opt)}(\mathbf{r})$, estimées par le filtre adapté matriciel pour les positions transverses x={-10;0;10} mm. La partie (d) de la figure 8 montre schématiquement une cartographie de l'orientation des fibres superposée à l'image échographique du mollet.

Au-delà de la détection et localisation de cibles, le filtre matriciel adapté peut être exploité à des fins d'imagerie quantitative des tissus en échographie ultrasonore. A titre d'exemple, il peut être réalisé une cartographie de l'anisotropie des tissus fibreux qui constituent typiquement les muscles. Cette mesure est particulièrement pertinente pour le diagnostic de maladies neuro-musculaires ou de maladies touchant le myocarde. En élastographie, les milieux fibreux génèrent également une anisotropie de vitesse des ondes de cisaillement. Une connaissance fine de cette anisotropie est donc requise pour cartographier de manière correcte la rigidité des tissus, à partir des films de propagation des ondes de cisaillement.

Cette troisième expérience a été réalisée sur un mollet, dont les fibres sont en partie visibles sur l'image échographique montrée sur la partie (a) de la figure 8. La matrice de réflexion $\mathbf{R}$ est enregistrée à l'aide d'un réseau linéaire de transducteurs (N = 256 éléments espacés de 0.2 mm, fréquence centrale 7.5 MHz et bande passante [2.5;9.5] MHz). La base d'illumination consiste en 101 ondes planes dont l'angle d'incidence varie de -25° à 25°. Pour chaque insonification, le champ réfléchi est enregistré par l'ensemble des transducteurs.

Pour imager quantitativement les tissus fibreux, le filtre matriciel adapté a été appliqué à la matrice $\mathbf{R}$ en considérant ici, comme objet de référence, un miroir de réflectivité constante dont les paramètres $\mathbf{q_i}$ sont ici l'orientation $\alpha$ par rapport à la sonde et la taille caractéristique $\ell_c$ (cf. la partie (b) de la figure 8). Un dictionnaire de matrices de référence $\mathbf{F}$ ($\mathbf{r}, \{\alpha, \ell_c\}$) associé à cet objet est construit à l'aide d'un calcul numérique décrit ci-dessous.

Le filtre matriciel adapté permet de calculer un indice de vraisemblance $P(\mathbf{r}, \{\alpha, \ell_c\})$ vis à vis des paramètres $\alpha$ et $\ell_c$ en chaque point $\mathbf{r}$ de l'image (cf. Eq. 10). La valeur maximale de cette quantité donne l'orientation $\alpha_c^{(opt)}(\mathbf{r})$ et la taille caractéristique $\ell_c^{(opt)}(\mathbf{r})$ des diffuseurs en chaque point de l'image :

[Math 11]

$$\left\{\alpha_c^{(opt)}(\mathbf{r}), \ell_c^{(opt)}(\mathbf{r})\right\} = \underset{\alpha, \ell_c}{\mathrm{argmax}}[P(\mathbf{r}, \{\alpha, \ell_c\})]$$

La taille et l'orientation des fibres ainsi déterminées sont représentées pour différentes positions transverses sur la partie (c) de la figure 8. Un seuil de détection a été appliqué *a priori* en ne représentant que le résultat du filtre matriciel dont l'indice de vraisemblance associé est supérieur à $10^{-3}$. La carte de l'orientation des fibres est également superposée à l'image échographique sur la partie (d) de la figure 8. Un très bon accord est trouvé entre l'aspect visuel des fibres sur l'image échographique et leur orientation déterminée par le filtre adapté matriciel.

Notons que cette approche peut être considéré comme une généralisation du beamforming spéculaire développé p.ex. par Rodriguez-Morales et al., cf. A. Rodriguez-Molares, A. Fatemi, L. Lovstakken and H. Torp, "Specular Beamforming," in IEEE Transactions on Ultrasonics, Ferroelectrics, and Frequency Control, vol. 64, no. 9, pp. 1285-1297, Sept. 2017, doi: 10.1109/TUFFC.2017.2709038.

Cette dernière approche correspond en effet à un filtre adapté pour un miroir de taille infinie. Le beamforming spéculaire est donc adéquat pour détecter et localiser de grands objets en échographie. En revanche, l'optimisation de la taille caractéristique du diffuseur permet d'avoir un filtre plus adapté aux milieux fibreux. C'est par ailleurs un paramètre important pour adapter localement la formation de voies aux différents types de fibres rencontrés et obtenir une image échographique de meilleure qualité. L'image formée est par exemple le maximum de l'observable *P* mesurée pour chaque pixel. En dehors de cela, d'autres objets peuvent être simulés de manière synthétique, par exemple une aiguille insérée dans le tissu.

Un lien existe entre le filtre matriciel adapté et l'analyse de la matrice de réflexion dans la base des ondes planes développée dans le cadre du contrôle non destructif à des fins de caractérisation des milieux anisotropes. Toutefois, cette approche ne permet pas de résoudre latéralement l'anisotropie des fibres ; seule l'évolution axiale de l'anisotropie peut être déterminée. La technique de corrélation spatiale du champ rétrodiffusé ne permet de déterminer l'orientation des fibres que dans un plan parallèle à la sonde.

La projection matricielle des données ultrasonores est une méthode très générale qui peut être appliquée à l'imagerie d'un nombre important de paramètres du milieu. Ici, des paramètres considérés sont par exemple la taille et l'orientation de diffuseurs, mais la forme ou la composition de ces derniers peuvent être également des paramètres que l'on peut cartographier avec cette méthode. Une condition nécessaire reste toutefois la constitution d'un dictionnaire suffisamment complet et précis des structures que l'on étudie. A cette fin, dans la section suivante, les différentes manières de procéder sont décrites pour constituer un tel dictionnaire.

**Constitution du dictionnaire de matrices de référence**

Il existe différentes manières de générer numériquement un dictionnaire des matrices de référence **F(r,q)**. Une première approche consiste à mesurer, lors d'une première étape de calibration, la matrice de réflexion $\mathbf{R_0(r_0,q)}$ associée à la cible seule, pour une position de cette dernière, puis à générer un dictionnaire de matrices de référence **F(r,q)** pour chaque point **r** du champ de vision. Une stratégie alternative consiste à calculer analytiquement l'ensemble des matrices de référence **F(r,q)**.

**Émulation numérique d'un dictionnaire à partir d'une mesure expérimentale**

La partie (a) de la figure 6 présente la configuration expérimentale pour la mesure de calibration de $\mathbf{R_0}$ réalisée dans le cadre de l'expérience de détection de cibles enfouies dans un milieu diffusant. Le réseau matriciel de 1024 transducteurs situé en *z*=0 est utilisé pour sonder, de manière indépendante, chaque cible placée à une position $\mathbf{r_0}$ et immergée dans l'eau. La matrice de référence associée, $\mathbf{R_{u\theta}^{(0)}(r_0,}\it{t})$, est enregistrée dans le domaine temporel entre la base des ondes planes en entrée ($\hat{\theta}_{in}$) et la base des transducteurs en sortie ($\mathbf{u}_{out}$). Elle contient ainsi l'ensemble des signaux mesurés par chacun des transducteurs repérés par leurs coordonnées $\mathbf{u}_{out}$ pour un ensemble d'illuminations en ondes planes se propageant dans la direction $\hat{\theta}_{in}$. Notons que ce choix de bases d'illumination et de réception peut être arbitraire et que la matrice de référence pourrait très bien être mesurée à l'aide d'une autre séquence d'illuminations, dans la base focalisée par exemple. En outre, des étapes intermédiaires peuvent être considérées, telle qu'un filtrage temporel de la matrix de référence mesurée, blanchiment du spectre, etc.

Une fois cette matrice de référence mesurée, une transformée de Fourier lui est appliquée dans le domaine temporel. Ainsi la matrice de référence, $\widetilde{\mathbf{R}}_{u\theta}^{(0)}(\mathbf{r_0},\it{f})$ dans le domaine fréquentiel est obtenue. Ensuite une translation de la cible peut être simulée numériquement à une nouvelle position r dans le milieu. Cette opération peut être effectuée dans différentes bases, par exemple la base focalisée. Une première étape consiste à projeter $\widetilde{\mathbf{R}}_{u\theta}^{(0)}(\mathbf{r_0},\it{f})$ dans cette base (Eq. [Math 3]) puis à générer une nouvelle matrice $\widetilde{\mathbf{R}}_{u\theta}^{(0)}(\mathbf{r},\it{f})$ pour une nouvelle position de cible r à partir des coefficients de $\widetilde{\mathbf{R}}_{rr}^{(0)}(\mathbf{r},\it{f})$, tel que :

[Math 12]

$$\tilde{R}_0(\mathbf{r}_{in},\mathbf{r}_{out},\mathbf{r}) = \tilde{R}_0(\mathbf{r}_{in} - \Delta\mathbf{r},\mathbf{r}_{out} - \Delta\mathbf{r},\mathbf{r_0})$$

avec $\Delta\mathbf{r} = \mathbf{r} - \mathbf{r_0}$.

Suivant la base d'acquisition de la matrice, on peut être également amené à travailler dans la base des transducteurs pour limiter le temps de calcul et/ou l'utilisation de mémoire. Une matrice $\widetilde{\mathbf{R}}_{uu}^{(0)}(\mathbf{r},\it{f})$ peut être générée

pour chaque position virtuelle **r** de la cible à partir de sa mesure en $\mathbf{r}_0$ en décomposant le déplacement $\Delta \mathbf{r}$ suivant ses projections transverse $\Delta\rho$ et axiale $\Delta z$. Le déplacement transverse se fait par une translation dans la base des transducteurs :

[Math 13]

$$\tilde{R}_0(\mathbf{u}_{\text{in}}, \mathbf{u}_{\text{out}}, \{\boldsymbol{\rho}, \mathbf{z_0}\}) = \tilde{R}_0(\mathbf{u}_{\text{in}} - \Delta\boldsymbol{\rho}, \mathbf{u}_{\text{out}} - \Delta\boldsymbol{\rho}, \{\boldsymbol{\rho_0}, \mathbf{z_0}\})$$

avec $\rho$ et $\rho_0$ les coordonnées transverses des points **r** et $\mathbf{r_0}$.

Le déplacement axial nécessite en revanche une propagation des données depuis le plan $z_0$ jusqu'au plan $z$ en entrée et sortie de la matrice $\widetilde{\mathbf{R}}_{\mathbf{uu}}^{(0)}$ :

[Math 14]

$$\widetilde{\mathbf{R}}_{\mathbf{uu}}^{(0)}(r) = \mathbf{G}_{\mathbf{u}\rho}(z) \times \mathbf{G}_{\mathbf{u}\rho}^{\dagger}(z_0) \times \widetilde{\mathbf{R}}_{\mathbf{uu}}^{(0)}(\{\boldsymbol{\rho}, z_0\}) \times \mathbf{G}_{\mathbf{u}\rho}^{*}(z_0)\mathbf{G}_{\mathbf{u}\rho}^{T}(z)$$

où le symbole T désigne l'opération de transposition matricielle.

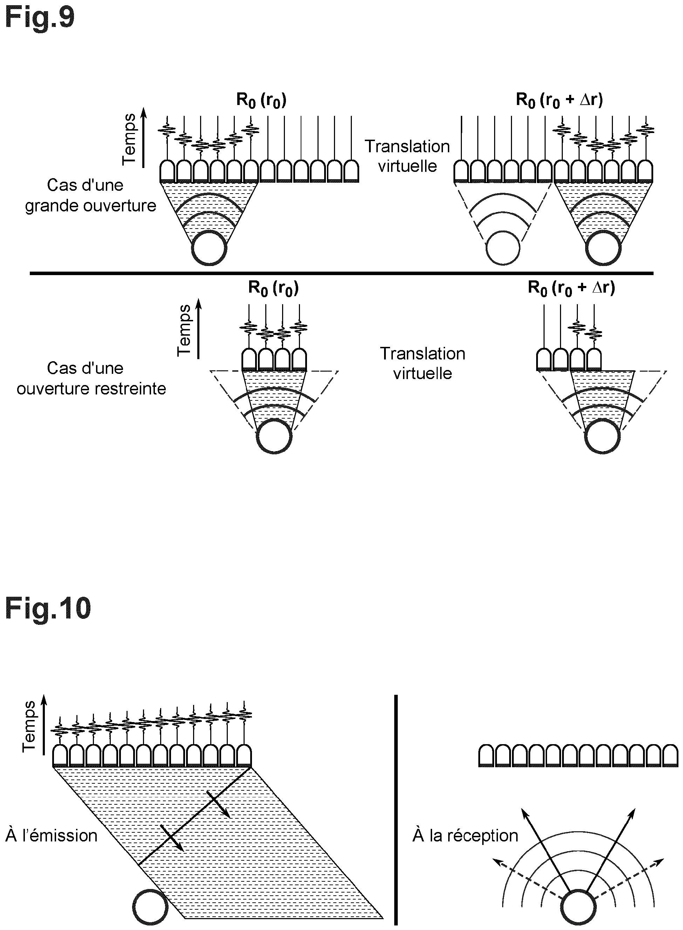

La figure 9 montre schématiquement la translation virtuelle de la position de l'objet cible selon des exemples de la présente divulgation. La partie haute (« Cas d'une grande ouverture ») de la figure 9 montre schématiquement qu'en effectuant cette translation dans la base des transducteurs ou dans la base focalisée, il est possible de déplacer virtuellement l'objet tant que le support du champ associé à la cible est contenu dans l'ouverture du réseau de transducteurs. La partie basse (« Cas d'une ouverture restreinte ») de la figure 9 montre schématiquement que pour une position latérale de la cible s'approchant des extrémités du réseau de transducteurs, une translation latérale des signaux ultrasonores n'est plus adéquate.

La matrice de réflexion $\mathbf{R}_0(\mathbf{r})$ peut être également générée à partir de la matrice $\mathbf{R}_0(\mathbf{r_0})$ depuis la base de Fourier. La première étape consiste donc à projeter la matrice $\mathbf{R}_0(\mathbf{r_0})$ acquise expérimentalement dans la base des ondes planes à la fois en entrée et sortie. Pour une matrice $\mathbf{R}_0(\mathbf{r_0})$ enregistrée à l'aide d'une séquence d'illumination en ondes planes, seule une projection en sortie dans la base de Fourier est requise :

[Math 15]

$$\widetilde{\mathbf{R}}_{\boldsymbol{\theta\theta}}^{(0)}(\mathbf{r_0}) = \mathbf{P}_{\boldsymbol{\theta}u} \times \widetilde{\mathbf{R}}_{\mathbf{u}\boldsymbol{\theta}}^{(0)}(\mathbf{r_0})$$

Dans le cas d'une matrice acquise dans la base canonique (transducteurs), la projection doit être effectuée à l'entrée et la sortie de la matrice : Il est à noter que la base canonique est seulement un exemple illustratif.

[Math 16]

$$\widetilde{\mathbf{R}}_{\boldsymbol{\theta\theta}}^{(0)}(\mathbf{r_0}) = \mathbf{P}_{\boldsymbol{\theta}u} \times \widetilde{\mathbf{R}}_{\mathbf{u}\boldsymbol{\theta}}^{(0)}(\mathbf{r_0})$$

Une fois la matrice $\tilde{R}_0(\mathbf{r_0})$ projetée dans la base des ondes planes, son déplacement virtuel à la position **r** peut être réalisé de la manière suivante :

[Math 17],

$$\tilde{R}(\hat{\theta}_{out}, \hat{\theta}_{in}, \mathbf{r}) = P(\hat{\theta}_{out}, \mathbf{r})P^{*}(\hat{\theta}_{out}, \mathbf{r_0})\tilde{R}(\hat{\theta}_{out}, \hat{\theta}_{in}, \mathbf{r})P^{*}(\hat{\theta}_{in}, \mathbf{r_0})P(\hat{\theta}_{in}, \mathbf{r})$$

$$= P(\hat{\theta}_{out}, \Delta\mathbf{r})\tilde{R}(\hat{\theta}_{out}, \hat{\theta}_{in}, \mathbf{r})P(\hat{\theta}_{in}, \Delta\mathbf{r})$$

Cette équation consiste à appliquer des rampes de phase en entrée et sortie aux données projetées dans la base des ondes planes. En fonction de la position **r** de l'objet, toutes les ondes planes émises depuis la sonde ne l'atteignent pas, de la même manière que les échos rétrodiffusés et arrivant jusqu'aux transducteurs ne peuvent provenir que d'une ouverture limitée (cf. la figure 10).

La figure 10 montre schématiquement la transmission et réception d'une onde plane associée à un angle d'incidence à partir duquel les données doivent être filtrées dans la matrice $\mathbf{R_0}$ selon des exemples de la présente divulgation. La partie gauche de la figure 10 montre schématiquement une onde plane associée à un angle d'incidence à partir duquel les données doivent être filtrées dans la matrice $\mathbf{R_0}$. La partie droite de la figure 10 montre schématiquement que à la réception, les ondes planes associées aux flèches pointant vers le réseau de transducteurs et les autres flèches pointant en dehors du réseau de transducteurs doivent être respectivement conservés et filtrés dans la matrice $\mathbf{R_0}$ mesurée.

Tout signal correspondant à des angles extrêmes, qu'il est possible de déterminer en fonction des géométries de la sonde et de la position de l'objet, est donc sans grand intérêt et peut être assimilé à du bruit, qu'il convient ainsi de filtrer dans une option d'implémentation.

Quelle que soit la base focalisée, celle des transducteurs ou dans la base de Fourier, les équations 12, 13 e 14 reposent sur une hypothèse d'invariance par translation du processus de focalisation (cf. la figure 9). Cette dernière hypothèse n'est vérifiée que si l'ensemble du champ associé à la cible est bien mesuré par le réseau de transducteurs :

[Math 18]

$$\sqrt{x^2 + y^2} < \frac{A}{2} - z \tan \beta$$

avec $\beta$, l'angle d'ouverture de la sonde imposé par sa directivité et A sa largeur.

Le choix de la base dans laquelle la translation virtuelle doit être réalisée est dicté par des considérations de temps et coûts de calculs. En mode quasi-temps-réel par exemple, on pourra choisir de réaliser toutes les opérations depuis la base d'acquisition des matrices de réflexion $\mathbf{R_0}$ et $\mathbf{R}$ afin de limiter le nombre d'opérations matricielles à réaliser.

## Optimisation de la matrice de référence

### Spécificité de la matrice de référence vis-à-vis du milieu environnant

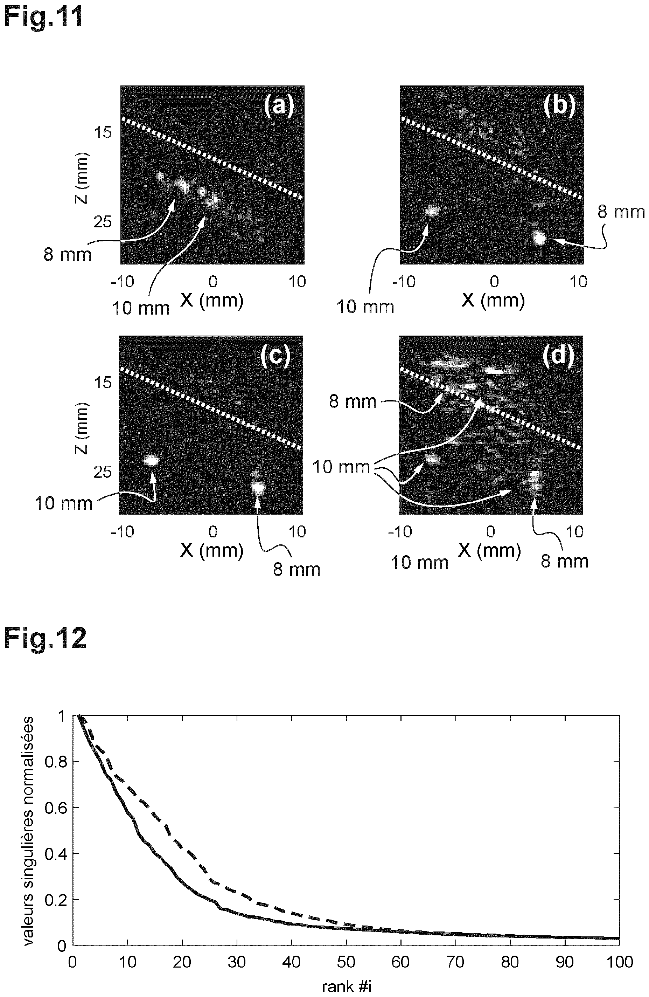

La figure 11 montre schématiquement des cartes de vraisemblance de position(s) pour chaque cible résultant de la projection matricielle définie par l'équation 10 selon des exemples de la présente divulgation. La partie (a) de la figure 11 montre schématiquement un filtre adapté avec $\tilde{\mathbf{F}}(\mathbf{r}, \varPhi) = \mathbf{R_0}(\mathbf{r}, \varPhi)$. La partie (b) de la figure 11 montre schématiquement un filtre adapté après suppression de l'écho spéculaire des cibles avec $\tilde{\mathbf{F}}(\mathbf{r}, \varPhi) = \mathrm{R_S}(\mathbf{r}, \varPhi)$ [cf. Eq. 19]. La partie (c) de la figure 11 montre schématiquement un filtre adapté avec $\tilde{\mathbf{F}}(\mathbf{r}, \varPhi) = \overline{\mathbf{R_S}}(\mathbf{r}, \varPhi)$ [cf. Eq. 20]. La partie (d) de la figure 11 montre schématiquement un filtre inverse avec $\tilde{\mathbf{F}}(\mathbf{r}, \varPhi) = \widehat{\mathbf{R}}_{\mathbf{S}}^{-1}(\mathbf{r}, \varPhi)$ [cf. Eq. 22]. Notons que le filtre en fréquence appliqué sur les données ultrasonores est ici large bande (fenêtre de Hann, [0;6] MHz).

Dans le cas de l'expérience de détection de cibles dans un milieu granulaire, une étape consiste à filtrer au préalable l'écho spéculaire des cibles dans $\mathbf{R_0}(\mathbf{r}, \varPhi)$. Sans ce filtrage préalable, l'image obtenue est dominée par l'écho d'interface du milieu granulaire (cf. la partie (a) de la figure 11). En effet, l'écho spéculaire n'est pas spécifique à la cible et va exalter également l'écho d'interface du milieu granulaire qui domine les signaux enregistrés dans la matrice **R.**

Il y a donc un intérêt à construire des matrices de référence $\mathbf{F}(\mathbf{r}, \varPhi)$ à partir des échos les plus spécifiques à chaque cible. Sur cet exemple illustratif, ce sont les échos de réflexions multiples en leur sein et les échos d'ondes de surface précédemment mis en lumière par la figure 6. En pratique, cette opération peut être réalisée par un fenêtrage temporel des signaux ultrasonores mesurés (cf. la partie (b) de la figure 6) :

[Math 19]

$$R_S(\mathbf{r_0}, \varPhi, t) = R_0(\mathbf{r_0}, \varPhi, t)H(t - t_0 - \delta t)$$

où H est la fonction de Heaviside, $t_0$ le temps d'arrivée de l'écho direct de la cible et $\delta t = \varDelta f^{-1}$ la résolution temporelle des signaux ultrasonores mesurés.

La matrice de référence spécifique $\mathbf{R_s}$ est ensuite translatée virtuellement et exploitée pour construire une image

$$\mathbf{F} = \mathbf{R}_S^{\dagger}$$

adaptée P($r$, $\Phi$) des cibles recherchées [cf. Eq. 9 avec    ]. Le résultat est dans cet exemple présenté sur la partie (c) de la figure 11. On note avantageusement que l'écho d'interface du milieu granulaire est beaucoup moins intense, les deux cibles sont détectées avec un bon contraste et la position de leur centre retrouvée avec une excellente précision.

### Blanchiment du spectre fréquentiel

Une seconde étape consiste à blanchir le spectre fréquentiel de la matrice $R_S$, tel que :

[Math 20]

$$\overline{\mathbf{R}}_s(\mathbf{r},\Phi,f) = \widetilde{\mathbf{R}}_s(\mathbf{r},\Phi,f)/\left\|\widetilde{\mathbf{R}}_s(\mathbf{r},\Phi,f)\right\|_2^{1/2}$$

En considérant cette famille de matrices normalisées $\overline{\mathbf{R}}_s$ comme dictionnaire de référence **F** dans l'équation 10, une nouvelle carte P($r$, $\Phi$) est obtenue sur la partie (c) de la figure 11. L'opération de blanchiment fréquentiel permet d'exploiter au mieux les degrés de liberté temporels mis en lumière par la partie (c) de la figure 6 et améliorer le contraste entre les cibles et le bruit associé au milieu environnant.

### Filtre inverse

Les précédents exemples ont été limités à un filtre adapté de la matrice **R** alors qu'un filtre inverse devrait pouvoir en théorie maximiser le contraste des deux cibles. Le processus d'inversion matricielle est mal conditionné et extrêmement sensible au bruit. Une méthode de régularisation peut être employée pour optimiser le processus d'inversion. A cette fin, la décomposition en valeurs singulières de $\overline{\mathbf{R}}_s$ doit être au préalable calculée :

[Math 21]

$$\overline{\mathbf{R}}_s(f) = \sum_{i=1}^{N} \sigma_i(f)\mathbf{U}_i(f)V_i^{\dagger}(f)$$

Le spectre des valeurs singulières est montré sur la figure 12 pour les deux cibles. Un grand nombre de valeurs singulières dépassent le niveau de bruit.

La figure 12 montre schématiquement un spectre des valeurs singulières $\sigma_i$ des matrices $\overline{\mathbf{R}}_s$ associées aux sphères selon des exemples de la présente divulgation, par exemple des sphères de diamètre $\Phi = 8$ mm et $\Phi = 10$ mm à la fréquence $f = 2.5$ MHz après fenêtrage temporel pour ne conserver que les échos de réflexions multiples et d'ondes de surface spécifiques à la cible.

L'ensemble des espaces propres associés à ces valeurs singulières forme le sous-espace signal associé à la cible. Le rang **Q** de ce sous-espace croît comme le nombre de cellules de résolution contenues dans la cible. Un estimateur $\widehat{\mathbf{R}}_S^{-1}(f)$ de la matrice inverse de $\widetilde{\mathbf{R}}_s(f)$ peut être calculé en inversant les **Q** premières valeurs singulières $\sigma_i$ du sous-espace signal de $\widetilde{\mathbf{R}}_s$ et en annulant celle du sous-espace bruit :

[Math 22]

$$\widehat{\mathbf{R}}_S^{-1}(f) = \sum_{i=1}^{Q} \sigma_i^{-1}(f)\mathbf{U}_i(f)V_i^{\dagger}(f)$$

Le dictionnaire résultant, $\widetilde{\mathbf{F}}(\mathbf{r},f) = \widehat{\mathbf{R}}_S^{-1}(\mathbf{r},f)$ , peut ensuite être utilisé pour construire une image de la probabilité de présence de la cible [cf. Eq. 10]. Le résultat est présenté sur la partie (d) de la figure 11 pour Q = 20. Certes, la résolution des échos associés à chaque cible est plus fine que pour le filtre adapté dans la partie (c) de la figure 11. Néanmoins, cette amélioration de la résolution se fait aux prix de fausses alarmes importantes en amont des cibles et d'un « cross-talk » (c'est-à-dire une l'interférence d'un premier signal avec un second) entre ces dernières. Ces artefacts sont liés à la sensibilité du processus d'inversion vis-à-vis du bruit expérimental.

Pour améliorer la robustesse de la méthode, dans une variante, une régularisation du type Tikhonov peut être appliquée. La matrice $\tilde{\mathbf{F}}(\mathbf{r_0}, f)$ s'exprime dans ce cas :

[Math 23]

$$\tilde{\mathbf{F}}(f) = \sum_{i=1}^{Q} \frac{\sigma_i(f)}{\sigma_i^2(f) + b^2} \mathbf{V}_i(f)\mathbf{U}_i^{\dagger}(f)$$

avec $b$, le niveau de bruit estimé sur le spectre des valeurs singulières.

**Génération numérique d'un dictionnaire**

Le dictionnaire de matrices de référence peut également être constitué de manière entièrement synthétique, *i.e* obtenu par calculs et simulation, sans mesure expérimentale. Pour illustrer, l'exemple de l'imagerie de l'anisotropie du muscle peut être pris comme exemple est repris. Les résultats montrés sur la figure 8 ont été obtenus en calculant numériquement la matrice de référence $\tilde{\mathbf{F}}(\mathbf{r_0})$ à partir du produit matriciel suivant :

[Math 24]

$$\widetilde{\mathbf{R}}_{uu}^{(0)}(\mathbf{r_0}) = \mathbf{P}_{\theta u}^{\dagger} \times \mathbf{P}_{\theta r} \times \Gamma(\mathbf{r}, \alpha, \ell_c) \times \mathbf{P}_{\theta r}^{T} \times \mathbf{P}_{\theta u}^{*}$$

où $\Gamma(\mathbf{r}, \alpha, \ell_c)$ représente la matrice de réflectivité d'un miroir plan d'inclinaison $\alpha$ et de taille $\ell_c$ [cf. la partie (b) de la figure 8] centré en $\mathbf{r}$ dans l'espace réel.
$\Gamma(\mathbf{r}, \alpha, \ell_c)$ est une matrice diagonale dont les coefficients $\gamma(\mathbf{r})$ correspondent à la réflectivité du miroir dont on simule la réponse.

En pratique, il est avantageux que l'espace réel soit échantillonné avec un pas de grille bien plus petit que la limite de diffraction ($\sim\lambda/5$). La grille définie dans l'espace réel n'est pas la même que celle de la base focalisée dont l'échantillonnage spatial est de l'ordre de $\lambda/2$.
Les résultats présentés sur la Figure 8 ont été obtenus à partir de l'équation 10 en considérant pour matrices de référence $\tilde{\mathbf{F}}(\mathbf{r}, \alpha, \ell_c)$ les matrices simulées $\mathbf{R_0}(\mathbf{r}, \alpha, \ell_c)$. Notons que, dans le cas d'un miroir plan, le nombre de valeurs singulières non nulles de $\mathbf{R_0}(\mathbf{r}, \alpha, \ell_c)$ est égal au nombre de cellules de résolution contenues dans l'objet et que ces valeurs propres sont toutes dégénérées. Le filtre inverse $\left[\tilde{\mathbf{F}}(\mathbf{r}, \alpha, \ell_c) = \widetilde{\mathbf{R}}_{\mathbf{0}}^{-1}(\mathbf{r}, \alpha, \ell_c)\right]$ donnerait donc un résultat strictement identique.
Tous ces modes de réalisations et d'autres exemples tels que décrits ci-dessus sont donnés uniquement à titre d'exemple non limitatif, et peuvent être combinés et/ou modifiés selon la portée de la présente divulgation.

**Revendications**

1. Procédé pour caractériser un objet cible dans un milieu, le procédé comprenant :

    obtenir une matrice de référence F(**q**) associée à l'objet cible,
    obtenir une matrice de réflexion **R** du milieu, et
    projeter la matrice de réflexion **R** sur la matrice de référence F(**q**) pour estimer une probabilité P(**q**) que l'objet soit dans un état **q**.

2. Procédé selon la revendication 1, dans lequel
    l'objet cible comprend au moins un parmi :

un élément prédéfini du milieu, tel qu'un tissu du milieu, et
un objet externe inséré dans le milieu, tel qu'un dispositif médical.

3. Procédé selon la revendication 1 ou 2, dans lequel
l'état q de l'objet cible comprend au moins un parmi :

la position de l'objet dans le milieu,
l'orientation de l'objet dans le milieu,
la forme et/ou la structure de l'objet,
la taille de l'objet,
la composition de l'objet, et
des paramètres locaux du milieu environnant, tels que la température, la pression, la composition, des propriétés mécaniques et/ou la concentration d'un composé du milieu environnant.

4. Procédé selon l'une quelconque des revendications précédentes, dans lequel, l'obtention de la matrice de réflexion comprend obtenir des matrices de réflexion mesurées à différents instants $t$, dans lequel
chacune des matrices de réflexion est projetée sur la matrice de référence pour mesurer la dynamique de l'état de l'objet.

5. Procédé selon l'une quelconque des revendications précédentes, dans lequel, l'obtention d'une matrice de référence $F(\mathbf{q})$ comprend obtenir un dictionnaire de matrices de référence $F(\mathbf{q})$ associé à l'objet cible, et
la projection de la matrice de réflexion $\mathbf{R}$ correspond à projeter la matrice de réflexion $\mathbf{R}$ sur l'ensemble des matrices de référence $F(\mathbf{q})$ pour estimer une probabilité $P(\mathbf{q})$ que l'objet soit dans un état $\mathbf{q}.$

6. Procédé selon l'une quelconque des revendications précédentes, dans lequel la projection de la matrice de réflexion $\mathbf{R}$ comprend l'application d'un filtre matriciel à la matrice de réflexion $\mathbf{R}.$

7. Procédé selon la revendication précédente, dans lequel

le filtre matriciel comprend un produit scalaire normalisé entre la matrice de réflexion $\mathbf{R}$ et l'ensemble des matrices de référence $\mathbf{F}(\mathbf{q})$, ou
le filtre matriciel comprend un modèle d'intelligence artificielle.

8. Procédé selon l'une quelconque des revendications précédentes, dans lequel les matrices de référence $\mathbf{F}(\mathbf{q})$ du dictionnaire dépendent d'un état $\mathbf{q}$ de l'objet cible, l'état optionnellement comprenant au moins un paramètre parmi : la position, l'orientation, la forme et/ou la structure, la taille, la composition.

9. Procédé selon l'une quelconque des revendications précédentes, dans lequel l'obtention de la matrice de référence $\mathbf{F}(\mathbf{q})$ comprend :

une mesure d'une première matrice de référence correspondant à une matrice de réflexion $\mathbf{R_0}(\mathbf{q_0})$ pour une première valeur $\mathbf{q_0}$ de l'état $\mathbf{q}$ de l'objet cible, et/ou
une mesure d'un premier jeu de matrices de référence associées à plusieurs états $\mathbf{q}$ de l'objet cible.

10. Procédé selon l'une quelconque des revendications précédentes, dans lequel l'obtention d'une matrice de référence $\mathbf{F}(\mathbf{q})$ comprend :
la génération numérique d'une deuxième matrice de référence pour un deuxième état $\mathbf{q}$ de l'objet cible à partir de d'une première matrice de référence mesurée pour un état $\mathbf{q_0}$ de l'objet cible.

11. Procédé selon la revendication précédente, dans lequel,
la deuxième matrice de référence est simulée numériquement à partir de la première matrice de référence par une translation de l'objet cible à un nouvel état $\mathbf{q}$ étant la position de l'objet, optionnellement dans une base focalisée, la base des transducteurs, ou la base d'ondes planes.

12. Procédé selon la revendication 9 ou 10, dans lequel
le dictionnaire de matrices de référence comprend la première et la deuxième matrices de référence.

13. Procédé selon la revendication 10, dans lequel

le dictionnaire des matrices de référence est optimisé en utilisant au moins l'une des méthodes suivantes :

adaptation du filtre matriciel en fonction de la première matrice de référence afin de rendre le filtre matriciel plus spécifique à l'objet cible recherché par rapport au milieu environnant ;

optimisation du spectre fréquentiel du dictionnaire pour améliorer le contraste entre l'objet cible et un bruit associé au milieu; et

construction du dictionnaire à partir d'un estimateur $\mathbf{R_0^{-1}}$ de la matrice inverse de la première matrice de référence.

14. Procédé selon l'une des revendications précédentes, dans lequel l'obtention de la première matrice de référence et/ou la matrice de réflexion comprend :

- une étape de génération d'une série d'ondes incidentes ($US_{in}$) dans un milieu, au moyen d'un réseau (10) de transducteurs (11), ladite série d'ondes incidentes étant une base d'émission ($\mathbf{i}$) ; et
- une étape de génération d'une matrice de réflexion expérimentale $\mathbf{R}_{ui}(t)$ définie entre la base d'émission ($\mathbf{i}$) en entrée et une base de réception ($\mathbf{u}$) en sortie ;
- une étape de détermination d'une matrice de réflexion focalisée $\mathbf{R_{rr}}=[R(\mathbf{r}_{in}, \mathbf{r}_{out}, \delta t)]$ dans une base focalisée, la matrice de réflexion focalisée $\mathbf{R_{rr}}$ comprend des réponses $R(\mathbf{r}_{in}, \mathbf{r}_{out}, \delta t)]$ du milieu entre un transducteur virtuel d'entrée ($TV_{in}$) de position spatiale $\mathbf{r}_{in}$ et un transducteur virtuel de sortie ($TV_{out}$) de position spatiale $\mathbf{r}_{out}$, les réponses du transducteur de virtuel de sortie ($TV_{out}$) étant prises à un instant temporel décalé d'un délai additionnel $\delta t$ par rapport à un instant temporel des réponses du transducteur virtuel d'entrée ($TV_{in}$), dans lequel

les ondes comprennent optionnellement des ondes ultrasonores.

15. Procédé selon l'une des revendications précédentes, dans lequel l'obtention d'une matrice de référence $F(\mathbf{q})$ comprend la constitution d'un dictionnaire de matrices de référence de manière entièrement synthétique en simulant numériquement :

une première matrice de référence d'un réflecteur virtuel pour un état $\mathbf{q_0}$ prédéfini, et
une deuxième matrice de référence pour un deuxième état $\mathbf{q}$ de l'objet cible,
optionnellement en fonction de la première matrice de référence $R_0(q)$.

# Fig.1

S1 : obtenir une matrice de référence F(q) ou un dictionnaire des matrices de référence associée à l'objet cible

S1a (option) : mesurer expérimentalement une première matrice de référence et/ou un premier jeu de matrices de référence

S1b (option) : constituer la matrice de référence F(q) ou le dictionnaire de matrices de référence de manière entièrement synthétique

S1c (option) : optimiser la matrice de référence F(q) ou le dictionnaire des matrices de référence

S2 : obtenir une matrice de réflexion R du milieu

S3 : projeter la matrice de réflexion R sur la matrice de référence F(q) pour estimer une probabilité P(q) que l'objet soit dans un état q

S3a (option) : appliquer un filtre matriciel à la matrice de réflexion R

# Fig.2

# Fig.3

**(a)** ÉMISSION

**(b)** RÉCEPTION

**(c)**

valeur singulière associée
à la première cible

valeur propre associée
à la seconde cible

**(d)**

**(e)**

# Fig.4

# Fig.5

Fig.6

# Fig.7

# Fig.8

# Fig.9

$R_0 (r_0)$

$R_0 (r_0 + \Delta r)$

Temps

Translation
virtuelle

Cas d'une
grande ouverture

$R_0 (r_0)$

$R_0 (r_0 + \Delta r)$

Temps

Cas d'une
ouverture restreinte

Translation
virtuelle

# Fig.10

Temps

À l'émission

À la réception

# Fig.11

# Fig.12

EP 4 574 056 A1

Europäisches Patentamt
European Patent Office
Office européen des brevets

**RAPPORT DE RECHERCHE EUROPEENNE**

Numéro de la demande

EP 24 22 1846

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | WILLIAM LAMBERT ET AL: "Reflection matrix approach for quantitative imaging of scattering media", ARXIV.ORG, CORNELL UNIVERSITY LIBRARY, 201 OLIN LIBRARY CORNELL UNIVERSITY ITHACA, NY 14853, 4 juin 2020 (2020-06-04), XP081680768, DOI: 10.1103/PHYSREVX.10.021048 * abrégé * * figures 1-6 * * Section I - VI * | 1-15 | INV. A61B8/08 |
| A | US 2016/317119 A1 (TAHMASEBI MARAGHOOSH AMIR MOHAMMAD [US] ET AL) 3 novembre 2016 (2016-11-03) * abrégé * * figures 1-5 * * alinéa [0009] - alinéa [0055] * | 1-15 | |
| A | WO 2022/229937 A1 (UNIV DO MINHO [PT]; INST POLITECNICO DO CAVADO E DO AVE [PT]) 3 novembre 2022 (2022-11-03) * abrégé * * figures 1-7 * * alinéa [0006] - alinéa [0089] * | 1-15 | DOMAINES TECHNIQUES RECHERCHES (IPC)<br><br>A61B |

Le présent rapport a été établi pour toutes les revendications

| Lieu de la recherche | Date d'achèvement de la recherche | Examinateur |
|---|---|---|
| Munich | 28 décembre 2024 | Moehrs, Sascha |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

# EP 4 574 056 A1

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-12-2024

| Document brevet cité au rapport de recherche | Date de publication | Membre(s) de la famille de brevet(s) | Date de publication |
|---|---|---|---|
| US 2016317119 A1 | 03-11-2016 | BR 112016013880 A2 | 08-08-2017 |
| | | CN 106061424 A | 26-10-2016 |
| | | EP 3086736 A1 | 02-11-2016 |
| | | EP 3542725 A1 | 25-09-2019 |
| | | JP 6483133 B2 | 13-03-2019 |
| | | JP 2016540604 A | 28-12-2016 |
| | | RU 2016129318 A | 25-01-2018 |
| | | US 2016317119 A1 | 03-11-2016 |
| | | WO 2015092667 A1 | 25-06-2015 |
| WO 2022229937 A1 | 03-11-2022 | EP 4329581 A1 | 06-03-2024 |
| | | US 2024216010 A1 | 04-07-2024 |
| | | WO 2022229937 A1 | 03-11-2022 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3114159

**Littérature non-brevet citée dans la description**

- **CLAIRE PRADA** ; **MATHIAS FINK**. Eigenmodes of the time reversai operator: A solution to sélective focusing in multiple-target media. *Wave Motion*, 1994, vol. 20 (2), ISSN 0165-2125, 151-163
- **MONTALDO G** ; **TANTER M** ; **BERCOFF J** ; **BENECH N** ; **FINK M.** Cohérent plane-wave compounding for very high frame rate ultrasonography and transient elastography.. *IEEE Trans Ultrason Ferroelectr Freq Control.*, March 2009, vol. 56 (3), 489-506

- **PAI, P.** ; **BOSCH, J.** ; **KÜHMAYER, M. et al.** Scattering invariant modes of light in complex media.. *Nat. Photonics*, 2021, vol. 15, 431-434
- **RODRIGUEZ-MORALES** ; **A. RODRIGUEZ-MOLA-RES** ; **A. FATEMI** ; **L. LOVSTAKKEN** ; **H. TORP et al.** Specular Beamforming. *IEEE Transactions on Ultrasonics, Ferroelectrics, and Frequency Control,*, September 2017, vol. 64 (9), 1285-1297